# EUROPEAN PATENT SPECIFICATION

(11) **EP 1 905 840 B1**
(45) Date of publication and mention of the grant of the patent: **22.12.2010**
(21) Application number: 07117132.6
(22) Date of filing: 25.09.2007
(51) Int. Cl.: C12Q 1/48, G01N 33/50

(54) **Method for assessing proliferation inhibiting effect of inhibitor, and method for screening a compound which inhibits proliferation of a tumor cell**
Verfahren zur Beurteilung der proliferationshemmenden Wirkung eines Hemmers und Verfahren zur Screening einer Verbindung zur Hemmung der Tumorproliferation
Procédé pour évaluer l'effet d'inhibition de la prolifération d'un inhibiteur, et procédé de criblage d' un composé inhibitant la prolifération des tumeurs

(30) Priority: 27.09.2006 JP 2006261699; 30.08.2007 JP 2007224923
(43) Date of publication of application: 02.04.2008
(73) Proprietor: SYSMEX CORPORATION, Kobe-shi, Hyogo 651-0073 (JP)
(72) Inventor: Notoya, Michitaka, Kobe-shi Hyogo 651-0073 (JP); Sato, Jun, Kobe-shi Hyogo 651-0073 (JP); Ohyama, Tomoko, Kobe-shi Hyogo 651-0073 (JP); Yoshida, Tomokazu, Kobe-shi Hyogo 651-0073 (JP); Ishihara, Hideki, Kobe-shi Hyogo 651-0073 (JP)
(74) Representative: De Clercq, Ann G. Y.

(56) References cited:
- EP-A- 1 840 221
- WO-A1-96/18626
- US-A1- 2002 187 511
- BOROWSKI PETER ET AL: "Protein kinase C-alpha produces reciprocal effects on the phorbol ester stimulated tyrosine phosphorylation of a 50 kDa kinase in Jurkat cells" BIOLOGICAL CHEMISTRY, vol. 380, no. 4, April 1999 (1999-04), pages 403-412, XP002467509 ISSN: 1431-6730
- BRAUN S ET AL: "Synthetic tyrosine polymers as substrates and inhibitors of tyrosine-specific protein kinases" JOURNAL OF BIOLOGICAL CHEMISTRY. (MICROFILMS), AMERICAN SOCIETY OF BIOLOGICAL CHEMISTS, BALTIMORE, MD, US, vol. 259, no. 4, 25 February 1984 (1984-02-25), pages 2051-2054, XP002436393
- DAVIS R J ET AL: "Amiloride directly inhibits growth factor receptor tyrosine kinase activity" JOURNAL OF BIOLOGICAL CHEMISTRY. (MICROFILMS), AMERICAN SOCIETY OF BIOLOGICAL CHEMISTS, BALTIMORE, MD, US, vol. 260, no. 4, 25 February 1985 (1985-02-25), pages 2543-2551, XP002436394
- SCHRAAG B. ET AL: "Standarization of an Enzyme-Linked immunosorbent Assay for the Determination of Protein Tyrosine Kinase Activity" ANALYTICAL BIOCHEMISTRY, vol. 211, 1993, pages 233-239, XP009007839
- QIN-YU XU ET AL: "Aggregation of IGF-I receptors or insulin receptors and activation of their kinase activity are simultaneously caused by the presence of polycations or K-ras basic peptides" BIOCHEMISTRY, vol. 30, 1991, pages 11811-11819,

## Description

### BACKGROUND OF THE INVENTION

### 1. Field of the Invention

The present invention relates to a method for assessing the proliferation inhibiting effect of a receptor tyrosine kinase inhibitor on a tumor cell, based on an activity value of receptor tyrosine kinases. Also, the present invention relates to a method for determining sensitivity of a tumor cell to receptor tyrosine kinase inhibitor, based on an activity value of receptor tyrosine kinases. Further, the present invention relates to a method for screening a compound, based on an activity value of receptor tyrosine kinases.

### 2. Description of the Related Art

Receptor tyrosine kinase present in a cell membrane (hereinafter, referred to as receptor tyrosine kinase) plays an important role in proliferation of a cell, cell survival, differentiation of a cell, and angiogenesis. For example, many of receptor tyrosine kinases are known to be associated with malignant alternation of a cell, and it is known that abnormality of an expression amount thereof or abnormality of the enzyme activity causes malignant alternation of a cell. Specifically, insulin-like growth factor receptor (IGFR) is reported to be overexpressed in a tumor cell. Furthermore, among human epithelial growth factor receptors (HERs), HER1 is known to be highly active mainly in a lung cancer, and HER2 is known to be highly active mainly in a breast cancer.

For the purpose of studying or treating a disease associated with receptor tyrosine kinase such as a cancer, an inhibitor which inhibits the activity of receptor tyrosine kinase has been studied and developed. For example, in the literature of N Osherov et al. (N Osherov et al., Selective inhibition of the epithelial growth factor and HER2/neu receptors by tryphostins, Journal of Biological Chemistry, May 1993, Vol. 268, p11134-11142), the ability of tyrphostin to inhibit HER1 and HER2 is studied.

As the first method of confirming the inhibiting ability described in the literature of N Osherov, there is a method of detecting autophosphorylation of HER1 or HER2. Specifically, a cell is lysed, and centrifuged to collect a membrane extract. This membrane extract and EGF are preincubated. Thereafter, the membrane extract and a reaction mixture containing [32P]-ATP and an inhibitor are mixed. After the resulting mixture was subjected to SDS-PAGE to separate a protein, a band of HER1 or HER2 incorporating 32P by autophosphorylation is detected by autoradiography.

In addition, in the literature of N Osherov et al., as a second method different from the first method, a method of detecting a phosphorylated substrate (Poly-Gu4-Tyr1) is described. In this method, a cell is lyzed, and centrifuged to collect a supernatant. After this supernatant and EGF are preincubated, HER1 or HER2 in the supernatant is immunoprecipitated using protein A Sepharose beads bound with an anti-HER1 extracellular domain monoclonal antibody. The precipitate obtained by immunoprecipitation, and a reaction mixture containing [32P] -ATP, an inhibitor, and Poly-Gu4-Tyr1 as a substrate are mixed, and incubated. After incubation, the precipitate is removed to obtain a supernatant. This supernatant contains Poly-Gu4-Tyrl phosphorylated with 32P. The supernatant is coated on a Whatman 3MM paper strip, and its radioactivity is measured to detect Poly-Gu4-Tyrl phosphorylated with 32P.

In the literature of N Osherov et al., the ability to inhibit HER1 and the ability to inhibit HER2 are confirmed, respectively, by such the methods.

US 2002/0187511 describes method for measuring a substance in solutions. The process makes it possible to determine the quantity of the substance, and to measure catalysed reactions which lead to the modification of the substance. The method makes it possible to test whether a substance modulates a catalysed reaction or not.

Borowski et al. (Borowski et al., Protein kinase C-α produces reciprocal effects on the phorbol ester stimulated tyrosine phosphorylation of a 50 kDa kinase in Jurkat cells, Biologican Chemistry, April 1999, Vol. 380, p403-412) describes an enhanced phosphorylation level of a detergent extract isolated from the enriched fraction of integral membrane proteins when phosphorylated in the presence of TPA and PDBu. Braun et al. (Braun et al., Synthetic tyrosine polymers as substrates and inhibitors of tyrosine-specific protein kinases, Journal of Biological Chemistry, Februari 1984, Vol. 259, p2051-2054) describes several synthetic polymers with ordered sequences which act as potent inhibitors of tyrosine-specific kinases. Specifically, cell membranes were isolated and the protein kinase activity was assayed using ATP, synthetic substrates and inhibitors.

Davis et al. (Davis et al., Amiloride directly inhibits growth factor receptor tyrosine kinase activity, Journal of Biological Chemistry, February 1985, Vol. 260, p2543-2551) describes that amiloride directly inhibits the tyrosine kinase activity of affinity-purified EGF, insulin and PDGF in vitro and can mediate such actions in vivo as demonstrated by phosphoamino acid analysis of total cell proteins.

EP 1 840 221 A1 describes a method for measuring the activity of a single receptor kinase present in a cell membrane of a cell.

Schraag et al. (Schraag et al., Standardization of an enzyme-linked immunosorbent assay for the determination of protein tyrosine kinase activity, Analytical Biochemistry, Vol. 211, p233-239) describes a procedure for an ELISA for the determination of protein tyrosine kinase activity from cytosolic and solubilized membrane fractions from breast cancers.

WO 96/18626 discloses inhibitors which are specific for EGF-receptor tyrosine kinase, in particular imidazole derivatives. A method is described for testing the inhibitory effect of such inhibitors.

For studying an inhibitor which is effective for treating a cancer, the proliferation inhibiting effect of an inhibitor on a tumor cell, and sensitivity of a tumor cell to an inhibitor are very useful information.

However, when the proliferation inhibiting effect of an inhibitor on a tumor cell, and sensitivity of a tumor cell to an inhibitor are examined, it is insufficient only to confirm the ability to inhibit individual receptor tyrosine kinases as described in the literature of N Osherov et al. This is because a plural kinds of receptor tyrosine kinases are present in a cell membrane of a tumor cell of a patient. Further, among receptor tyrosine kinases, some are activated by formation of a heterodimer. In addition, there is a possibility that unknown receptor tyrosine kinases or mutants are present in a cell membrane of a tumor cell. Further, an inhibitor dose not necessarily act on only one kind of receptor tyrosine kinase. From the foregoing, it is desired to develop the technique which can examine the proliferation inhibiting effect of an inhibitor on a tumor cell, and sensitivity of a tumor cell to an inhibitor, based on influence of an inhibitor on various kinds of receptor tyrosine kinases of a tumor cell.

### SUMMARY OF THE INVENTION

The scope of the present invention is defined solely by the appended claims, and is not affected to any degree by the statements within this summary.

A first aspect of the present invention is a method for assessing the proliferation inhibiting effect of a receptor tyrosine kinase inhibitor, an a tumor cell, by determining the activity value of the group of receptor tyrosine kinases in a sample of said tumor cell in accordance with appended claims 1 to 7.

A further aspect of the present invention is a method for screening a compound which inhibits proliferation of a tumor cell in accordance with appended claims 8 to 11. ,

### BRIEF DESCRIPTION OF THE DRAWINGS

Fig. 1 is the detection result of phosphorylation of a GST-poly (Glu, Tyr) substrate by receptor tyrosine kinase.
Fig. 2 is a view showing a structural formula of each inhibitor used in the present embodiment.
Fig. 3 is a view showing the result of Example 1.
Fig. 4 is a view showing the result of Comparative Example 1.
Fig. 5 is a view showing the result of Comparative Example 2.
Fig. 6 is a view showing the result of Example 2.
Fig. 7 is a view showing the result of Comparative Example 3.

### DESCRIPTION OF THE PREFERRED EMBODIMENTS

In the method of the present embodiment, a cytoplasm is separated from a tumor cell to prepare a sample containing receptor tyrosine kinases. Since a various kinds of receptor tyrosine kinases are present in a cell membrane of a cell, a group of receptor tyrosine kinases derived from a cell membrane are contained in a sample obtained by separating a cytoplasm from a cell. According to the invention, the prepared sample is separated into a first and a second sample. Then, the first sample is treated with an inhibitor which inhibits the activity of receptor tyrosine kinases, And, receptor tyrosine kinases in the first and second sample, and universal substrates of receptor tyrosine kinases are contacted. Thereby, the substrate may be phosphorylated by the activity of receptor tyrosine kinases in the first and second sample. And, the phosphorylated substrate is detected and, based on the detection result, an activity value of receptor tyrosine kinases in the first and second sample is measured. The thus obtained activity value reflects influence of the inhibitor on the various kinds of receptor tyrosine kinases present in the cell, and it is correlated with the effect of inhibiting proliferation of the cell by the inhibitor. Therefore, when an activity value is measured using a tumor cell in the aforementioned method, it is possible to assess the proliferation inhibiting effect of the inhibitor on the tumor cell based on the difference between the first and second activity values of the group of receptor tyrosine kinases. It can be envisaged that, when an activity value is measured using a tumor cell in the aforementioned method, it is possible to determine sensitivity of the tumor cell to the inhibitor based on the resulting activity value. And, the thus obtained assessment result of the proliferation inhibiting effect of the inhibitor on the tumor cell, and determination result of sensitivity of the tumor cell to the inhibitor are very useful information for studying a receptor tyrosine kinase inhibitor which is effective in treating a cancer.

The receptor tyrosine kinase is not particularly limited as far as it is tyrosine kinase present in a cell membrane. Specific examples include growth factor receptors such as insulin receptor (IR), insulin-like growth factor receptor (IGFR), platelet-derived growth factor receptor (PDGFR), fibroblast growth factor (FGFR), human epithelial growth factor receptor (HER), and vascular endothelial growth factor (VEGFR). The HER family includes HER1, HER2, HER3 and HER4.

Receptor tyrosine kinase is obtained from a cell membrane of a tumor cell. The cell may be a cell contained in a biological sample collected from a biological body, or a cultured cell obtained by establishing a cell collected from a biological body. Specifically, as the cell, a tumor cell is used. And, the tumor cell may be a tumor cell contained in a biological sample collected from a patient, or a cultured cell obtained by establishing a tumor cell collected from a patient.

A method of obtaining receptor tyrosine kinases is not particularly limited as far as it is a method of separating a cytoplasm from a cell to prepare a sample containing a plurality of receptor tyrosine kinases. For example, receptor tyrosine kinases can be obtained by the following method. First, a cell membrane of a cell is fragmentated in a suitable buffer solution (hereinafter, referred to as homogenization reagent). The resulting solution is separated into a supernatant and a precipitate by centrifugation, and the supernatant is removed. This supernatant contains proteins derived from a cytoplasm. The precipitate contains a fragment of a cell membrane retaining a variety of receptor tyrosine kinases. This precipitate and a solution containing a surfactant (hereinafter, referred to as solubilizing reagent) are mixed. The resulting mixed solution is separated into a supernatant and a precipitate by centrifugation. The supernatant contains a cell membrane containing a variety of receptor tyrosine kinases, which may be micellized with a surfactant. The precipitate contains insoluble proteins and DNAs. And, this supernatant can be used as a sample containing receptor tyrosine kinases. In the thus prepared sample, a cell membrane may be micellized with a surfactant, in the state where a variety of receptor tyrosine kinases may be penetrated through a fragmented cell membrane.

It is known that receptor tyrosine kinase forms a homodimer or a heterodimer by binding with a ligand. The sample prepared by the aforementioned method contains receptor tyrosine kinase in the state where a steric structure to such an extent that a homodimer or a heterodimer can be formed, is retained. Therefore, by using the sample prepared by the aforementioned method, it is possible to assess the proliferation inhibiting effect of an inhibitor on a tumor cell, or determine sensitivity of a tumor cell to an inhibitor more precisely.

The homogenization reagent is used in order to prevent receptor tyrosine kinase from denaturing upon fragmentation of a cell. A pH of the homogenization reagent is not particularly limited as far as it is in such a range that receptor tyrosine kinase can be obtained in the stable state without denaturing or inactivating it. Such the pH is preferably 4.0 to 9.0, more preferably 4.5 to 8.5, further preferably 5.0 to 8.0. It is preferable that the homogenization reagent contains a buffer. Examples of the buffer include a phosphate buffer, an acetate buffer, a citrate buffer, MOPS (3-morpholinopropanesulfonic acid), HEPES (2-[4-(2-hydroxyethyl)-1-piperazinyl]ethanesulfonic acid), Tris (tris(hydroxymethyl)aminomethane), Tricine (N-[tris(hydroxymethyl)methyl]glycine) and the like. It is preferable that the solubilizing reagent contains the aforementioned buffer, and has a similar extent of a pH to that of the homogenization reagent.

And, a protease inhibitor, a dephosphorylase inhibitor, a reagent for preventing oxidation of a SH group (hereinafter, referred to as thiol group stabilization agent) or the like may be added to the homogenization reagent and/or the solubilizing reagent.

A method of fragmentating a cell membrane is not particularly limited as far as it can fragmentate a cell membrane. Examples include suction discharge with a pipette, stirring with a vortex mixer, grinding with a blender, pressurization with a pestle, ultrasound treatment with a ultrasound treating apparatus and the like.

A surfactant contained in the solubilizing reagent can be used for solubilizing (micellizing) a fragmentated cell membrane. It is preferable to use a surfactant which does not degrade or denature receptor tyrosine kinase contained in a cell membrane. A surfactant having a charge may bind to receptor tyrosine kinase and change the steric structure of the receptor tyrosine kinase. For this reason, it is preferable to use a nonionic surfactant which does not substantially bind to receptor tyrosine kinase. Examples of such the nonionic surfactant include nonionic surfactants having, as a fundamental structure, dodecyl ether, cetyl ether, stearyl ether, p-t-octylphenyl ether or the like. Specific examples include Nonidet P-40 (NP-40, registered trademark of Shell International Petroleum Company Limited), Triton X (registered trademark of Union Carbide Chemicals and Plastics Inc.), Tween (registered trademark of ICI Americas Inc.), Brij (registered trademark of ICI Americas Inc.), Emulgen (registered trademark of Kao Corporation) and the like. A concentration of the surfactant in the solubilizing reagent is preferably 0.05 to 5%, more preferably 0.1 to 3%, further preferably 0.1 to 1%.

The protease inhibitor can be used in order to prevent receptor tyrosine kinase from being degraded with a protease contained in a cell. Examples of the protease inhibitor include metalloprotease inhibitors such as EDTA and EGTA, serine protease inhibitors such as PMSF, trypsin inhibitor and chymotrypsin, cysteine protease inhibitors such as iodoacetamide, and E-64, and the like. These protease inhibitors may be used alone, or by mixing them. Alternatively, a commercially availably product in which a plurality of protease inhibitors have been mixed in advance, such as protease inhibitor cocktail (Sigma) may be used.

The dephosphorylase inhibitor can be used in order to prevent the enzyme activity of receptor tyrosine kinase from being reduced with dephosphorylase contained in a cell. Examples of the dephosphorylase inhibitor include sodium orthovanadate (Na₃VO₄), sodium fluoride (NaF), and okadaic acid. Dephosphorylation inhibitors may be used alone, or by mixing a plurality of them.

The thiol group stabilizing agent can be used in order to prevent inactivation of receptor tyrosine kinase. A thiol group contained in an enzyme is oxidized, and easily forms a more stable disulfide. Formation of disulfide may be cause for inactivation of an enzyme in some cases since it changes a structure of the enzyme. Oxidation of the thiol group can be prevented with a reagent containing a thiol group. Examples of the thiol group stabilizing agent include dithiothreitol (DTT), 2-mercaptoethanol, glutathione, cysteine, homocysteine, coenzyme A, dihydrolipoic acid and the like. For example, when the thiol group stabilizing agent is DTT, a concentration of the thiol group stabilizing agent in the homogenization reagent and/or the solubilizing reagent is preferably 0.05 to 2 mM, more preferably 0.07 to 1.7 mM, further preferably 0.1 to 1.5 mM. For example, when the thiol group stabilizing agent is 2-mercaptoethanol, a concentration of the thiol group stabilizing agent in the homogenization reagent and/or the solubilizing reagent is preferably 0.1 to 15 mM, more preferably 0.3 to 13 mM, further preferably 0.5 to 12 mM.

In the present embodiment, a cytoplasm is separated from a tumor cell to prepare a sample containing a plurality of receptor tyrosine kinases, and the prepared sample is treated with an inhibitor.

The inhibitor is not particularly limited as far as it inhibits the activity of receptor tyrosine kinase. As the inhibitor of receptor tyrosine kinase, there are inhibitors which bind to an ATP-binding site of receptor tyrosine kinase, inhibitors which bind to a substrate-binding site of receptor tyrosine kinase, and inhibitors which bind to an extracellular domain (e.g. ligand-binding site) of receptor tyrosine kinase. Examples of the inhibitor which binds to an ATP-binding site of receptor tyrosine kinase (hereinafter, referred to as ATP-competitive tyrosine kinase inhibitor) include Iressa (AstraZeneca), Glyvec (Novartis Pharma), Tarceva (OSI), PD153035 (Calbiochem), AG1478 (Calbiochem), 4557W (EGFR/ErbB-2 Inhibitor) (Calbiochem), PDGF Receptor Tyrosine Kinase Inhibitor III (Calbiochem), and VEGF Receptor Tyrosine Kinase Inhibitor III (Calbiochem). Examples of the inhibitor which binds to a substrate-binding site of receptor tyrosine kinase include tyrphostin (Calbiochem). Examples of the inhibitor which binds to an extracellular domain of receptor tyrosine kinase include Herceptin (Genentech), Cetuximab (ImClone), and pertuzumab (Genentech).

Treatment of the sample with the inhibitor is not particularly limited as far as it is treatment which can contact the aforementioned inhibitor and receptor tyrosine kinase in a sample. Examples of such a treating method include dissolution of the inhibitor in a suitable solution such as a buffer solution, and mixing of this solution with a sample.

In the present embodiment, an activity value of receptor tyrosine kinases in a first sample treated with the inhibitor and a second sample inhibitor is measured.

In the method of measuring an activity value of receptor tyrosine kinases, a universal substrate of receptor tyrosine kinases is utilized. Specifically, receptor tyrosine kinase contained in a sample, and a universal substrate of receptor tyrosine kinases are contacted. By this contact, the substrate is phosphorylated with the activity of receptor tyrosine kinases in the treated sample. And, the phosphorylated substrate is detected and, based on the resulting detection result, an activity value can be determined. Preferably, the sample treated with the inhibitor, and a universal substrate of receptor tyrosine kinases, and a phosphate group donor are mixed to contact them. And, the substrate phosphorylated with the activity of receptor tyrosine kinases is detected. Receptor tyrosine kinase is activated by autophosphorylation. Receptor tyrosine kinase activated by autophosphorylation then phosphorylates the substrate. However, even receptor tyrosine kinase is autophosphorylated, the substrate is not necessarily phosphorylated. Therefore, detection of phosphorylation of the substrate can determine an activity value more precisely.

It can be envisaged that, as the substrate of receptor tyrosine kinases, a mixture of a plural kinds of substrates respectively having high specificity for particular receptor tyrosine kinase can be used. The mixture comprises a plural kinds of substrates with different specificity for receptor tyrosine kinase. As the substrate having high specificity for particular receptor tyrosine kinase, for example, Grb2 , myelin basic protein (MBP), histone H2B (HH2B), phospholipase C gamma, which are a substrate having high specificity for HER1, can be used. Alternatively, a commercially available substrate such as GST-EGFR substrate (Stratagene) may be used as the substrate having high specificity for HER1. The GST-EGFR substrate is a fused protein of GST, and a substrate artificially prepared so that it can be phosphorylated by the enzyme activity of HER1. By using the mixture of different substrates in measurement, it may be possible to measure an activity value of receptor tyrosine kinase consisting of various receptor tyrosine kinases in a sample.

According to the invention, as the substrate of receptor tyrosine kinases, a universal substrate for receptor tyrosine kinases is used wherein the universal substrate comprises a poly (Glu:Tyr) (4:1) peptide, a poly (Glu:Tyr) (1:1) peptide, a poly (Glu:Tyr:Ala) (6:1:3) peptide, a poly (Glu:Tyr:Ala) (1:1:1) peptide, or a poly (Glu:Tyr:Ala:Lys) (2:1:6:5) peptide. For example, the universal substrate is a common substrate for each of receptor tyrosine kinases. The universal substrate can be phosphorylated with a first receptor tyrosine kinase, a second receptor tyrosine kinase which is different from the first receptor tyrosine kinase, a third receptor tyrosine kinase which is different from the first and second receptor tyrosine kinase, and a fourth receptor tyrosine kinase which is different from the first, second and third receptor tyrosine kinase. In other words, the universal substrate may be a substrate which can be substantively phosphorylated with various receptor tyrosine kinases. Examples of the universal substrate include the known synthetic peptide artificially prepared so that the peptide can be phosphorylated with receptor tyrosine kinase regardless of a kind of receptor tyrosine kinase. Examples include synthetic peptides which are used as a substrate for tyrosine kinase, in the literature of Norio Sakai et al., (Norio Sakai et al., 1985, The Journal of Biological Chemistry, Vol.260, No.17, 9793-9804), the literature of Sergei Braun et al., (Sergei Braun et al., 1984, The Journal of Biological Chemistry, Vol.259, No.4, 2051-2054), and the literature of M.Abdel-Ghany et al., (M.Abdel-Ghany et al., 1990, Proceeding of The National Academy of Science, Vo1.87, 7061-7065) and the like. Synthetic peptides disclosed in these literatures consist of an amino acid sequence comprising glutamic acid (hereinafter, abbreviated as Glu) and tyrosine residue (hereinafter, abbreviated as Tyr), and are artificially prepared so that Tyr can be phosphorylated with two or more kinds of tyrosine kinases. Examples of the amino acid sequence include following amino acid sequences.

An amino acid sequence in which a sequence consisting of four Glus and one Tyr is repeated two or more times (hereinafter, referred to as amino acid sequence "a"),
an amino acid sequence in which a sequence consisting of one Glu and one Tyr is repeated two or more times (hereinafter, referred to as amino acid sequence "b"),
an amino acid sequence in which a sequence consisting of six Glus, one Tyr and three alanine residues (hereinafter, abbreviated as Ala) is repeated two or more times (hereinafter, referred to as amino acid sequence "c"),
an amino acid sequence in which a sequence consisting of one Glu, one Tyr and one Ala is repeated two or more times (hereinafter, referred to as amino acid sequence "d"),
an amino acid sequence in which a sequence consisting of two Glus, one Tyr, six Alas and five lysine residues (hereinafter, abbreviated as Lys) is repeated two or more times (hereinafter, referred to as amino acid sequence "e").

In addition, in the literature of Hunter (Tony Hunter, 1982, The Journal of Biological Chemistry, Vol.257, No.9, 4843-4848), there is a report that an acidic amino acid residue is important in phosphorylating Tyr with tyrosine kinase. Thereby, particularly, an amino acid sequence "a" and an amino acid sequence "c" containing many Glus which are an acidic amino acid residue are preferable. By using a substrate consisting of such the amino acid sequence in measurement, it may be possible to measure an activity value of receptor tyrosine kinase consisting of various receptor tyrosine kinases contained in a sample.

In order to assess the proliferation inhibiting effect of an inhibitor on a tumor cell, and determine sensitivity of a tumor cell to an inhibitor, more precisely, it is preferable to measure an activity value of receptor tyrosine kinase consisting of as many kinds as possible of receptor tyrosine kinases of the tumor cell. Therefore, according to the invention the aforementioned universal substrate is used.

In the enzyme reaction which is catalyzed by receptor tyrosine kinase, a phosphate group of a phosphate group donor is taken into a substrate with the enzyme activity of receptor tyrosine kinase activated by autophosphorylation. Examples of the phosphate group donor include adenosine triphosphate (ATP), adenosine 5'-O-(3-thiotriphosphate) (ATP-γS), 32P-labeled adenosine 5'-O-(3-triphosphate) (γ-[32P]-ATP), adenosine diphosphate (ADP), adenosine monophosphate (AMP) and the like.

It is preferable that the substrate has an affinity tag. Using the affinity tag and a solid phase having a binding substance which can bind to the affinity tag (hereinafter, referred to as solid phase), the substrate can be recovered. Specifically, a complex in which the substrate having the affinity tag and the solid phase are bound is recovered, and binding of the affinity tag in the recovered complex, and a binding substance possessed by the solid phase is dissociated, thereby, the substrate can be finally recovered.

The affinity tag is not particularly limited as far as it is a substance which can bind to a binding substance, and does not hamper binding of the substrate to receptor tyrosine kinase, and phosphorylation of the substrate. As the affinity tag, for example, polypeptide, hapten and the like can be used. Specifically, as the affinity tag, glutathione-S-transferase (hereinafter, referred to as GST), histidine, maltose-binding protein, FLAG peptide (Sigma), Myc tag, HA tag, Strep tag (IBA GmbH), biotin, avidin, streptavidin and the like can be used.

As the substrate having the affinity tag, for example, a fused protein of a substrate and an affinity tag can be used. As the fused protein, an entity in which an affinity tag and a substrate are bound may be used. Alternatively, a vector having a recombinant gene expressing a fused protein of an affinity tag and a substrate is introduced into a host, and a fused protein produced by the host may be used.

The binding substance is not particularly limited as far as it can dissociably bind to the affinity tag. Examples of the binding substance include glutathione, nickel, amylose, FLAG antibody (Sigma), Myc antibody, hemagglutinin (HA) antibody, Strep-Tactin (IBA GmbH) and the like.

The solid phase is not particularly limited as far as it is a carrier which can bind to the binding substance. Examples of a material for the solid phase include polysaccharides, plastics, glasses and the like. Examples of a form of the solid phase include beads, gel and the like. Examples of the solid phase include Sepharose beads, agarose beads, magnetic beads, glass beads, silicone gel and the like. Alternatively, the aforementioned beads and gel may be used by filling into a column.

Examples of the combination of the affinity tag and the solid phase include the following examples.

When GST is selected as the affinity tag, as the solid phase, for example, glutathione Sepharose beads (hereinafter, referred to as glutathione beads) can be used. In that case, phosphorylated GST-substrate and glutathione beads are bound with the enzyme activity of receptor tyrosine kinase. Glutathione beads bound with GST-substrate are recovered and, when reductive glutathione is added to the recovered glutathione beads, binding between GST and glutathione beads can be dissociated. Thereby, the phosphorylated GST-substrate can be recovered.

Upon recovery of the phosphorylated GST-EGFR substrate, the GST-substrate and glutathione beads are bound in advance, which may be used in an enzyme reaction, or after an enzyme reaction, the GST-substrate and glutathione beads may be bound.

In addition, when histidine is selected as the affinity tag, as the solid phase, for example, nickel agarose beads can be used. Binding of histidine and nickel can be dissociated, for example, using an acid such as Glycine-HCI and the like, or imidazole.

When a maltose-binding protein is selected as the affinity tag, as the solid phase, for example, amylose magnetic beads can be used. Binding of the maltose-binding protein and amylose can be dissociated, for example, by adding free amylose.

When the FLAG peptide is selected as the affinity tag, as the solid phase, the FLAG affinity gel of Sigma can be used. Binding of the FLAG peptide and the FLAG affinity gel can be dissociated, for example, by using an acid such as Glycine-HCl and the like, or 3×FLAG peptide (Sigma).

When the Myc tag is selected as the affinity tag, as the solid phase, for example, agarose beads bound with a Myc antibody can be used. In addition, when the HA tag is selected as the affinity tag, agarose beads bound with a HA antibody can be used. Both of binding of the Myc tag and the Myc antibody, and binding of the HA tag and the HA antibody can be dissociated, for example, by adding an acid or an alkali to denature a protein. Thereupon, it is preferable to select an acid or an alkali which can revert the denatured protein to the original state. Specifically, examples of the acid include hydrochloric acid and the like, and examples of the alkali include sodium hydroxide.

When the Strep tag is selected as the affinity tag, as the solid phase, a Strep-Tactin solid-phased gel column of IBA GmbH can be used. Binding of the Strep tag and the Strep-Tactin can be dissociated using desthiobiotin which irreversibly reacts with streptavidin.

After receptor tyrosine kinases in a sample and a substrate are enzyme-reacted, and before the substrate is recovered, the enzyme reaction may be stopped using heat treatment, cooling treatment, or EDTA. In a step of recovering the substrate, the enzyme reaction further proceeds in some cases, and this may be cause for generation of scatter of measurement result in every sample. However, by stopping the enzyme reaction before recovery of the substrate, this can be avoided.

For detecting a phosphorylation substrate, a labeling substance is used. Examples of the labeling substance are not limited to, but include a fluorescent substance, an enzyme, a radioactive isotope and the like. Examples of the fluorescent substance include fluorescein, coumarin, eosin, phenanthroline, pyrene, rhodamine and the like. Examples of the enzyme include alkaline phosphatase, peroxidase and the like. Examples of the radioactive isotope include 32P, 33P, 131I, 125I, 3H, 14C, 35S and the like.

For detecting a phosphorylation substrate, a labeling substance is bound to the phosphorylation substrate. For example, by using an antibody which has a labeling substance and can specifically bind to a phosphorylation substrate, the labeling substance can be bind to the phosphorylation substrate.

Alternatively, by using an antibody which can specifically bind to a phosphorylation substrate (hereinafter, referred to as phosphorylating substrate-specific antibody), and an antibody which can bind to a phosphorylating substrate-specific antibody and has a labeling substance (hereinafter, referred to as secondary antibody), the labeling substance can be bound to the phosphorylation substrate. In this case, the labeling substance can be substantially bound to the phosphorylation substrate via the phosphorylating substrate-specific antibody and the secondary antibody.

Alternatively, by using the phosphorylating substrate-specific antibody, a secondary antibody having biotin, and avidin having a labeling substance, the labeling substance can be bound to the phosphorylation substrate. In this case, the labeling substance can be substantially bound to the phosphorylation substrate via the phosphorylating substrate-specific antibody, the secondary antibody, biotin and avidin. Alternatively, the secondary antibody may have avidin, and biotin may have the labeling substance.

Alternatively, a phosphorylating substrate-specific antibody having biotin, and avidin having a labeling substance may be used. Alternatively, a phosphorylating substrate-specific antibody having avidin, and biotin having a labeling substance may be used.

By detecting the labeling substance, the phosphorylated substrate can be detected, thereby, finally the activity of receptor tyrosine kinases can be measured.

As the aforementioned phosphorylating substrate-specific antibody and secondary antibody, an antibody obtained by contacting an animal with an antibody to promote immunity, and purifying blood of the animal, an antibody obtained by gene recombination, a polyclonal antibody, a monoclonal antibody and the like can be used. Alternatively, a mixture of at least two kinds of these antibodies may be used. The antibody as used herein includes a fragment of an antibody, and a derivative thereof. Examples of the antibody include a Fab fragment, a F (ab') fragment, a F(ab)2 fragment, a sFv fragment and the like (Blazar et al., 1997, Journal of Immunology, 159: 5821-5833 and Bird et al., 1988, Science, 242: 423-426). As a class of the antibody, IgG, IgM and the like can be used, being not limiting.

A method of detecting the phosphorylation substrate is appropriately selected depending on a kind of the labeling substance. When the labeling substance is a fluorescent substance, phosphorylation of a substrate can be detected by Western blotting. The phosphorylated substance is separated with a membrane, the phosphorylating substrate-specific antibody is added to bind to the phosphorylation substrate, and a secondary antibody having a fluorescent substance is bound to the phosphorylating substrate-specific antibody, and this fluorescence may be detected. When the phosphorylation substrate is separated in advance using the aforementioned affinity tag, phosphorylation of the substrate may be detected using a slot blot method in place of Western blotting. As the labeling substance, an enzyme may be used in place of the fluorescent substance. When the enzyme is used, the enzyme possessed by a secondary antibody is subjected to a color developing reaction by adding a substrate, and this color development may be detected.

Alternatively, a solution containing the phosphorylation substrate is accommodated in a tube, a phosphorylating substrate-specific antibody having a fluorescent substance is added to bind to the phosphorylated substrate, and a fluorescent intensify is measured, thereby, phosphorylation of the substrate may be detected.

When the labeling substance is an enzyme, phosphorylation of a substrate can be detected by solid phase enzyme-linked immunosorbent assay (hereinafter, referred to as ELISA method). The ELISA method includes direct adsorption method and sandwich method.

In the direct adsorption method, a phosphorylation substrate is adsorbed onto a surface of a solid phase, a phosphorylating substrate-specific antibody having an enzyme is added to bind to the phosphorylated substrate. Then, an enzyme possessed by the phosphorylating substrate-specific antibody is subjected to a color developing reaction by adding a substrate, and this color development may be detected.

In the sandwich method, a phosphorylating substrate-specific antibody is bound to a solid phase (hereinafter, referred to as solid-phased antibody), and a phosphorylation substrate is added to a solid-phased antibody. Then, a phosphorylating substrate-specific antibody having an enzyme (hereinafter, referred to as labeled antibody) is added to bind to the phosphorylated substrate. An enzyme possessed by the labeled antibody is subjected to a color developing reaction by adding a substrate, and this color development may be detected.

For example, when the enzyme is alkaline phosphatase, a mixed solution of nitroblue tetrazolium chloride (NBT) as a substrate, and 5-bromo-4-chloro-3-indoxyl phosphate (BCIP) are used to react them, thereby, a color can be developed. When the enzyme is peroxidase, diaminobenzidine (DAB) as a substrate is used to react them, thereby, a color can be developed.

When the sandwich method is used, it is preferable that the solid-phased antibody and the labeled antibody are bound to different sites of the phosphorylated substrate. That is, it is preferable that there are a plurality of antibody-binding sites in the phosphorylated substrate, or two kinds of antibodies used recognize different determinants of the phosphorylated substrate.

When the labeling substance is a radioactive isotope, phosphorylation of a substrate can be determined by radioimmunoassay (hereinafter, referred to as RIA). Specifically, a phosphorylating substrate-specific antibody having a radioactive isotope is bound to a phosphorylated substrate, radiation is measured with a scintillation counter or the like, thereby, phosphorylation of a substrate can be detected.

Like this, in the method of the present embodiment, an activity value of receptor tyrosine kinase consisting of a variety of receptor tyrosine kinases in a sample is measured. And, the resulting activity value reflects influence of an inhibitor on a variety of kinds of receptor tyrosine kinases present in a cell, and is correlated with the effect of inhibiting proliferation of a cell by an inhibitor. Therefore, when an activity value is measured using a tumor cell in the aforementioned method, it may be possible to assess the proliferation inhibiting effect of an inhibitor on the tumor cell based on the resulting activity value. It can be envisaged that, when an activity value is measured using a tumor cell in the aforementioned method, it may be possible to determine sensitivity of the tumor cell to an inhibitor based on the resulting activity value.

The proliferation inhibiting effect of an inhibitor on a tumor cell can be assessed based on an activity value of receptor tyrosine kinases in a sample treated with an inhibitor, which is obtained by the aforementioned method. It can be envisaged that, by comparing the resulting activity value with a predetermined threshold, the proliferation inhibiting effect can be assessed. For example, when the resulting activity value is less than a threshold, it may be judged that the proliferation inhibiting effect is high. When the resulting activity value is not less than a threshold, it may be judged that the proliferation inhibiting effect is low.

According to the invention, a first activity value of receptor tyrosine kinases contained in a sample treated with an inhibitor, and a second activity value of receptor tyrosine kinases in a sample not treated with the inhibitor are measured, and the proliferation inhibiting effect can be assessed based on the resulting first activity value and second activity value. Specifically, for example, when the first activity value and the second activity value are compared, and significant decrease in the first activity value is recognized, it may be judged that the proliferation inhibiting effect is high. When significant decrease in the first activity value is not recognized, it may be judged that the proliferation inhibiting effect is low. Significant decrease in the first activity value can be confirmed, for example, by calculating a difference or a proportion between the first activity value and the second activity value, and comparing the resulting difference or proportion with a predetermined threshold. For example, when a difference between the first activity value and the second activity value is not less than a threshold, it may be determined that significant decrease in the first activity value is recognized, and it may be judged that the proliferation inhibiting effect is high. When the difference is less than a threshold, it may be determined that significant decrease in the first activity value is not recognized, and it may be judged that the proliferation inhibiting effect is low. And, when a proportion of the first activity value and the second activity value is less than a threshold, it may be determined that significant decrease in the first activity value is recognized, and it may be judged that the proliferation inhibiting effect is high. When the proportion is not less than a threshold, it may be not determined that significant decrease in the first activity value is not recognized, and it may be judged that the proliferation inhibiting effect is low.

Similarly, it can be envisaged that sensitivity of a tumor cell to an inhibitor can be determined based on an activity value of receptor tyrosine kinases in a sample treated with an inhibitor, which is obtained by the aforementioned method. Specifically, by comparing the resulting activity value and a predetermined threshold, sensitivity can be assessed. For example, when the resulting activity value is less than a threshold, it may be determined that a tumor cell is sensitive to an inhibitor. When the resulting activity value is not less than a threshold, it may be determined that a tumor cell is not sensitive to an inhibitor.

In addition, a first activity value of receptor tyrosine kinases in a sample treated with an inhibitor, and a second activity value of receptor tyrosine kinases in a sample not treated with the inhibitor are measured, and sensitivity can be determined based on the resulting first activity value and second activity value. Specifically, for example, when the first activity value and the second activity value are compared, and significant decrease in a first activity value is recognized, it may be assessed that a tumor cell is sensitive to the inhibitor. When significant decrease in the first activity value is not recognized, it may be determined that a tumor cell is not sensitive to the inhibitor. Significant decrease in the first activity value can be confirmed, for example, by calculating a difference or a proportion between the first activity value and the second activity value, and comparing the resulting difference or proportion with a predetermined threshold. For example, when a difference between the first activity value and the second activity value is not less than a threshold, it may be determined that significant decrease in the first activity is recognized, and it may be determined that a tumor cell is sensitive to the inhibitor. When the difference is less than a threshold, it may be determined that significant decrease in the first activity value is not recognized, and it may be determined that a tumor cell is not sensitive to the inhibitor. And, when a proportion between the first activity value and the second activity value is less than a threshold, it may be determined that significant decrease in the first activity value is recognized, and it may be determined that a tumor cell is sensitive to the inhibitor. When the proportion is not less than a threshold, it may be determined that significant decrease in the first activity value is not recognized, and it may be determined that a tumor cell is not sensitive to the inhibitor.

The activity value obtained by the aforementioned method reflects influence of an inhibitor on a variety of receptor tyrosine kinases present in a cell, and is correlated with the effect of inhibiting proliferation of the cell by the inhibitor. Therefore, from another viewpoint, it can be envisaged that utilizing the method of the present embodiment, a compound which inhibits the activity of receptor tyrosine kinases of a tumor cell can be screened. Specifically, a cytoplasm is separated from a tumor cell to prepare a sample containing receptor tyrosine kinases. The resulting sample is treated with a candidate compound. By contacting receptor tyrosine kinases in the treated sample, and a substrate for at least two kinds of receptor tyrosine kinases, the substrate is phosphorylated by the activity of receptor tyrosine kinases in the treated sample. The phosphorylated substrate is detected, and an activity value of receptor tyrosine kinases in the treated sample is determined based on the detection result. And, based on the resulting activity value, a compound which inhibits the activity of receptor tyrosine kinases present in the cell membrane of the tumor cell can be screened. In an aspect of the invention, based on an activity value obtained as in the aforementioned screening method, a compound which inhibits proliferation of a tumor cell can be screened. As described above, it is known that abnormality of an expression amount or enzyme activity of receptor tyrosine kinases causes malignant alternation of a cell. Further, an inhibitor of receptor tyrosine kinase such as Iressa is utilized as an anti-cancer agent. From these things, the aforementioned screening method can be utilized in screening a prospective compound in study of generating a drug of an anti-cancer agent.

It can be envisaged that reagents used in the aforementioned measurement can be formulated into a reagent kit. This kit comprises a substrate for receptor tyrosine kinase, a phosphate group donor containing a phosphate group which can be introduced into a substrate by the activity of receptor tyrosine kinase, and a labeling substance which can bind to a substrate with a phosphate group introduced therein, and emits a detectable signal. The above components may be accommodated into a single container, or at least one component may be accommodated into another container. Preferably, a reagent containing the substrate and the phosphate group donor are accommodated into a first container, and a reagent containing the labeling substance is accommodated into a second container. In addition, when the labeling substance consists of a primary antibody which can bind to a substrate, and a secondary antibody which can bind to a primary antibody and has a labeling substance, it is preferable that these primary antibody and secondary antibody are accommodated into separate containers. When a sample is treated with an inhibitor in measurement, the reagent may contain the inhibitor. The reagent may contain a buffer for adjusting a pH. As the buffer, the aforementioned buffers can be used. The reagent kit may comprise a homogenization reagent and/or a solubilizing reagent.

The method of the present invention will be explained in more specifically below based on Examples. The present invention is not limited to these Examples.

In the following Examples, a universal substrate for a plural kinds of receptor tyrosine kinases was used. First, a method of preparing the substrate will be explained.

### 1. Preparation of a substrate for tyrosine kinase

A fused protein of a peptide consisting of an amino acid sequence (SEQ ID No.:1) in which a sequence consisting of four glutamic acid residues and one tyrosine residue is repeated five times (hereinafter, referred to as poly(Glu, Tyr)peptide), and GST was prepared. The prepared fused protein was used as a substrate which can be phosphorylated with receptor tyrosine kinase regardless of a kind of receptor tyrosine kinase. Hereinafter, this fused protein is referred to as GST-poly (Glu, Tyr) substrate.

The GST-poly(Glu, Tyr) substrate was prepared by the following method. Using a DNA (SEQ ID No.:2) encoding an amino acid sequence (SEQ ID No.:1) of the poly (Glu, Tyr), a sense primer (SEQ ID No.:3) and an antisense primer (SEQ ID No.:4) designed based on a nucleotide sequence of this DNA, as well as KODplusDNA polymerase (Toyobo Co., Ltd.), PCR was performed. The amplification product obtained by PCR (hereinafter, referred to as poly(Glu, Tyr)DNA), and pGEX-4T-3 (GE Healthcare Bioscience) which is a plasmid vector for expressing the GST fused protein were treated with a restriction enzyme (BamH1 and EcoR1) . And, the poly(Glu, Tyr)DNA was incorporated into the pGEX-4T-3 to prepare a recombinant plasmid. This recombinant plasmid was transformed into Escherichia coli JM109. The resulting Escherichia coli was cultured in a liquid medium (LB medium) until an absorbance (600 nm) of a culturing solution became 0.6. To the cultured Escherichia coli was added 1 mM IPTG (concentration in culturing solution), and this was cultured for 4 hours to induce expression. Then, Escherichia coli was lysed, and the GST-poly(Glu, Tyr) substrate was recovered using glutathione Sepharose 4B (GE Healthcare Bioscience). Amino acid sequence of this GST-poly(Glu, Tyr) is shown in SEQ ID No.5.

Then, it was confirmed that the prepared GST-poly (Gly, Tyr) substrate is phosphorylated with a variety of receptor tyrosine kinases.

### 2. Detection of phosphorylation of GST-poly(Glu, Tyr) substrate

Using an intracellular domain (ICD) of commercially available receptor tyrosine kinase, the GST-poly(Gly, Tyr)substrate was phosphorylated. By Western blotting, the phophorylated GST-poly(Gly, Tyr)substrate was detected. And, receptor tyrosine kinase is composed of an extracellular domain, a transmembrane domain, and an intracellular domain, and a site exhibiting the activity of tyrosine kinase is present in an intracellular domain.

### (Preparation of sample for reaction)

Fifty microliter of a buffer 1 (containing 20 mM HEPES pH7 . 4, 10 mM MnCl2, 1%NP40, 1 mM DTT, 0.2% protease inhibitor (hereinafter, referred to as PI), 10% glycerol, 200 µM Na₃ VO₄ and 50 mM NaF), and 0.5 pmol of ICD of commercially available receptor tyrosine kinase were mixed. The resulting mixed solution was used as a sample for reaction in the following enzyme reaction. Herein, as ICD, PDGF Receptor β Kinase (hereinafter, referred to as PDGFR-β), VEGF Receptor 1 Kinase (hereinafter, referred to as VEGFR1), VEGF Receptor 2 Kinase (hereinafter, referred to as VEGFR2), EGF Receptor 1 Kinase (hereinafter, referred to as HER1), ErbB2 Kinase (hereinafter, referred to as HER2), ErbB4 Kinase (hereinafter, referred to as HER4), and IGF-1Receptor Kinase (hereinafter, referred to as IGF1R) (all Cell Signaling Technology) were used. And, a mixture of the buffer 1 and the PDGFR-β was used as a sample for reaction i. A mixture of the buffer 1 and the VEGFR1 was used as a sample for reaction ii. A mixture of the buffer 1 and the VEGFR2 was used as a sample for reaction iii. A mixture of the buffer 1 and the HER1 was used as a sample for reaction iv. A mixture of the buffer 1 and the HER2 was used as a sample for a reaction v. A mixture of the buffer 1 and the HER3 was used as a sample for reaction vi. A mixture of the buffer 1 and the IGF1R was used as a sample for reaction vii.

### (Enzyme reaction)

Twenty five microliter of a sample for reaction i, and 25 µl of a substrate solution 1 containing a GST-poly(Glu, Tyr)substrate (containing 20 mM HEPES pH7.4, 10 mM MnCl₂, 1 mM DTT, 1% NP40, 0.2% PI, 10% glycerol, 200 µM Na₃VO₄, 50 mM NaF, 40 µM ATP, and 5µg GST-poly (Glu, Tyr) substrate) were mixed, and incubated at 25°C for 60 minutes. To this reaction solution was added 25 µl of a SDS sample buffer pH6.8 (containing 200 mM Tris, 40% glycerol, 8% SDS, and 10% 2-mercaptoethanol), and this was boiled at 100°C for 5 minutes to stop an enzyme reaction. The thus prepared solution is designated as sample for SDS i(+). Similarly, samples for SDS ii(+) to vii(+) were prepared from samples for reaction ii to vii.

Separately, 25 µl of a sample for reaction i, and 25 µl of a substrate solution 2 not containing ATP (containing 20 mM HEPES pH7.4, 10 mM MnCl₂, 1 mM DTT, 1% NP40, 0.2% PI, 10% glycerol, 200 µM Na₃VO₄, 50 mM NaF, and 5 µg GST-poly(Glu, Tyr) substrate) were mixed, and incubated at 25°C for 60 minutes. To this reaction solution was added 25 µl of a SDS sample buffer, and this was boiled at 100°C for 5 minutes to stop an enzyme reaction. The thus prepared solution is designated as sample for SDS i (-). Similarly, samples for SDS ii (-) to vii (-) were prepared from samples for reaction ii to vii. The substrate solution 2 has the same composition as that of the substrate solution 1 except that ATP is not contained. And, samples for SDS i (-) to vii (-) were used as a negative control of samples for SDS i (+) to vii(+).

### (Detection of phosphorylated GST-poly(Glu, Tyr) substrate)

Respective samples for SDS were injected into separate wells of a polyacrylamide gel (PAG mini "primary" 4/20(13W) (Daiichi Pure Chemicals Co., Ltd.)), and electrophoresed at 25 mA for 70 minutes using an electrophoresis bath (cassette electrophoresis bath "primary" DPE-1020 (mini duplicate) (Daiichi Pure Chemicals Co., Ltd.)). By applying a voltage at 100V for 1 hour using a mini transblotting cell (Bio-rad), proteins separated by electrophoresis were transferred from a polyacrylamide gel to a polyvinylidene fluoride (PVDF) membrane (Immobilon-FL 0.45 µm pore size (Millipore)). This PVDF membrane was blocked with a 4% Block Ace (Dainippon Sumitomo Pharma Co., Ltd.) solution. The blocked PVDF membrane was shaken in 2 ml of a primary antibody solution (containing 0.4% Block Ace and 0.5 µg/ml Anti-Phosphotyrosine clone 4 G10 (upstate)) for 60 minutes, and washed with TBS-T (containing 25 Mm Tris, 150 mM NaCl and 0.1% Tween-20) three times. Then, this PVDF membrane was shaken in 2 ml of a secondary antibody solution (containing 0.4% Block Ace and 2.7 µg/ml Anti-mouse immunoglobulin•rabbit polyclonal antibody FITC label (DAKO)) for 60 minutes, and washed with TBS-T three times. This PVDF membrane was dried, and analyzed using an image analyzing apparatus (Pharos FX system (Bio-rad)) to detected fluorescence. Like this, by Western blotting, phosphorylated GST-poly (Glu, Tyr) substrates contained in samples for SDS i (+) to vii (+) and samples for SDS i (-) to vii (-) were detected.

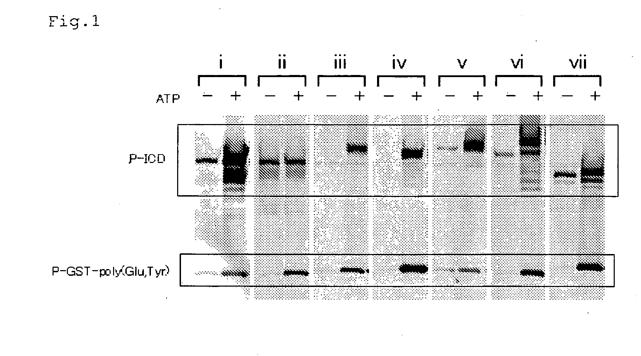

Fig. 1 is a fluorescence photograph showing the result of Western blotting. In the figure, i shows the result in the case of use of PDGFR-β, ii shows the result in the case of use of VEGFR1, iii shows the result in the case of use of VEGFER2, iv shows the result in the case of use of HER1, v shows the result in the case of use of HER2, vi shows the result in the case of use of HER4, and vii shows the result in the case of use of IGF1R. In addition, in respective photographs of i to vii, - is the result obtained from the sample for SDS prepared using the substrate solution 2 not containing ATP. + is the result obtained from the sample for SDS prepared using the substrate solution 1 containing ATP. P-ICD shows a position where autophosphorylated tyrosine kinase appears and P-GST-poly (Glu, Tyr) shows a position where the phosphorylated GST-poly (Glu, Tyr) substrate appears.

At + of all (i to vii) of Fig. 1, a single band was seen at positions where the phosphorylated GST-poly (Glu, Tyr) substrate appears. Thereby, it was seen that the GST-poly (Glu, Tyr) substrate is phosphorylated with various kinds of receptor tyrosine kinases.

In addition, at - of ii, iii, iv, vi and vii of Fig. 1, no band was seen at positions where the phosphorylated GST-poly (Glu, Tyr) substrate appears. It is thought that this is because, in the enzyme reaction, ATP is not contained in the reaction solution, and the GST-poly (Glu, Tyr) substrate was not phosphorylated. On the other hand, at - of i and v of Fig. 1, a very faint band was seen at positions where the phosphorylated GST-poly (Glu, Tyr) substrate appears. It is thought that this is because an antibody used in detection non-specifically bound or because a minor amount of ATP was mixed into a product used in measurement.

### Example 1

### Influence of ATP competitive tyrosine kinase inhibitor

In the present Example, a sample for reaction containing various receptor tyrosine kinases was prepared from a cultured cell. The prepared sample for reaction was treated with an ATP competitive tyrosine kinase inhibitor. And, using a GST-poly (Glu, Tyr) substrate, an activity value of a receptor tyrosine kinase group contained in the treated sample for reaction was measured. Based on the obtained activity value, an extent that the inhibitor inhibits the receptor tyrosine kinase activity of a cultured cell was studied.

### (Sample for reaction)

From five kinds of cultured cells (MDA-MB453, MDA-MB468, SKBr3, Hela and HT29), a sample for reaction was prepared. Specifically, the cultured cell and 1 ml of a cell treating solution (containing 20 mM HEPES pH7.4, 0.2% PI, 10% glycerol, 200 µM Na₃VO₄, and 50 mM NaF) were mixed. By pressing using a pestle, a cell membrane of the resulting mixed solution was destructed to prepare a cell solution. The resulting cell solution was centrifuged, a supernatant was discarded, and a precipitate was recovered. The recovered precipitate and a cell membrane solubilizing solution (containing 20 mM HEPES pH7.4, 1% NP40, 0.2% PI, 10% glycerol, 200 µM Na₃VO₄, and 50 mM NaF) were mixed. A cell membrane in the mixed solution obtained by pressing using a pestle was solubilized, and centrifuged to recover a supernatant. This supernatant was used as a sample for reaction. MDA-MB453 is a cultured cell derived from a breast cancer, and a sample for reaction prepared from this cell is designated as sample for reaction MB453. MDA-MB468 is a cultured cell derived from a breast cancer, and a sample for reaction prepared from this cell is designated as sample for reaction MB468. SKBr3 is a cultured cell derived from a breast cancer, and a sample for reaction prepared from this cell is designated as sample for reaction SKBr3. Hela is a cultured cell derived from a uterine cervical cancer, and a sample for reaction prepared from this cell is designated sample for reaction Hela. HT29 is a cultured cell derived from a large intestine cancer, and a sample for reaction prepared from this cell is designated sample for reaction HT29.

### (Binding of GST-poly (Glu, Tyr) substrate to EILSA plate)

As a plate for ELISA, a glutathione-coated plate (Reacti-Bind Clear Glutathione Coated Plates, 8-well Strip (PIERCE)) was used. First, each well of the plate was washed with TBS-T (containing 25 mM Tris, 150 mM NaCl and 0.05% Tween 20) three times. Then, into each well was placed 50 µl of the substrate solution 1 containing the GST-poly (Glu, Tyr) substrate prepared in the 1 (TBS containing 5 µg/ml GST-poly (Glu, Tyr) substrate), and this was incubated at 25°C for 1 hour while slightly shaken. After incubation, each well was washed with TBS-T two times. Further, each well was washed with 20 mM HEPES pH7.4 (containing 0.05% Tween 20) once. Like this, the GST-poly (Glu, Tyr) substrate was bound to a surface of a well of the plate for ELISA. This plate for ELISA was used in the following enzyme reaction.

### (Treatment with ATP competitive tyrosine kinase inhibitor)

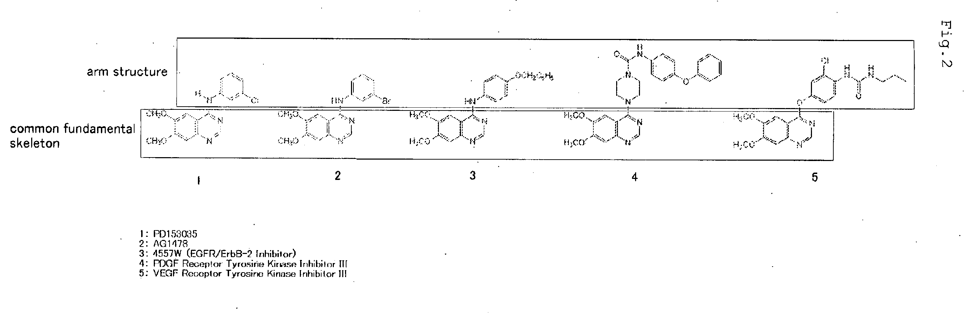

In the present Example, five kinds of ATP competitive tyrosine kinase inhibitors of PD153035 (Calbiochem), AG1478 (Calbiochem), 4557W (EGFR/ErbB-2 Inhibitor) (Calbiochem), PDGF Receptor Tyrosine Kinase Inhibitor III (Calbiochem) and VEGF Receptor Tyrosine Kinase Inhibitor III (Calbiochem) were used. PD153035 is an inhibitor of HER1 and HER2. AG1478 is an inhibitor of HER1 and HER2. 4557W is an inhibitor of HER1 and HER2. PDGF Receptor Tyrosine Kinase Inhibitor III is an inhibitor of PDGFR. VEGF Receptor Tyrosine Kinase Inhibitor III is an inhibitor of VEGFR. A structural formula of each inhibitor is shown in Fig. 2.

First, 25 µl of the sample for reaction MB453 was dispensed into 6 tubes, respectively. Among 6 tubes, to the first tube was added 25 µl of a treating solution containing 400 µM (20 mM HEPES pH7.4, 20 mM MnCl₂, 2 mM DTT, 1% NP40, 10% glycerol, 200 µM Na₃VO₄, 50 mM NaF, 400 µM ATP). To the second tube was added 25 µl of a treating solution containing 400 µM AG1478. To the third tube was added 25 µl of a treating solution containing 400 µM 4557W. To the fourth tube was added 25 µl of a treating solution containing 400 µM PDGF Receptor Tyrosine Kinase Inhibitor III. To the fifth tube was added 25 µl of a treating solution containing 400 µM VEGF Receptor Tyrosine Kinase Inhibitor III. To the sixth tube was added 25 µl of a treating solution containing no inhibitor. The thus obtained solutions are used as a reaction solution. Similarly, using the sample for reaction MB468, the sample for reaction SKBr3, the sample for reaction Hela and the sample for reaction HT29, samples for reaction were prepared. In order to suppress the enzyme activity of receptor tyrosine kinase, this working was performed under the condition of not higher than 4°C.

### (Enzyme reaction and detection of phosphorylated substrate)

Fifty microliter of each reaction solution was placed into separate wells of the plate for ELISA, and this was incubated at 25°C for about 30 minutes. After incubation, 100 µl of a reaction stopping solution (TBS-T containing 1 mM EDTA) was added to each well, followed by further washing with TBS-T three times. Then, each well was washed with 300 µl of StartingBlock T20 (TBS) Blocking Buffer (PIERCE). A HRP-labeled primary antibody (p-Tyr(PY20), sc-508 HRP(SANTA Cruz Biotechnology)) was 1000-fold diluted with StartingBlock T20 (TBS) Blocking Buffer to prepare a primary antibody solution. One hundred microliter of the prepared primary antibody solution was placed into each well after washing, and incubated at 25°C for about 1 hour and 30 minutes while slightly shaken. After incubation, each well was washed with TBS-T five times. After washing, 150 µl of a TMB solution (3,3',5,5'-Tetramethylbenzidine (TMB) Liquid Substrate System for ELISA (Sigma)) was placed into each well, a color was developed adequately at room temperature for 5 to 30 minutes while light was shielded, and an absorbance (650 nm) was measured with VersaMax (Molecular Device).

### (Result)

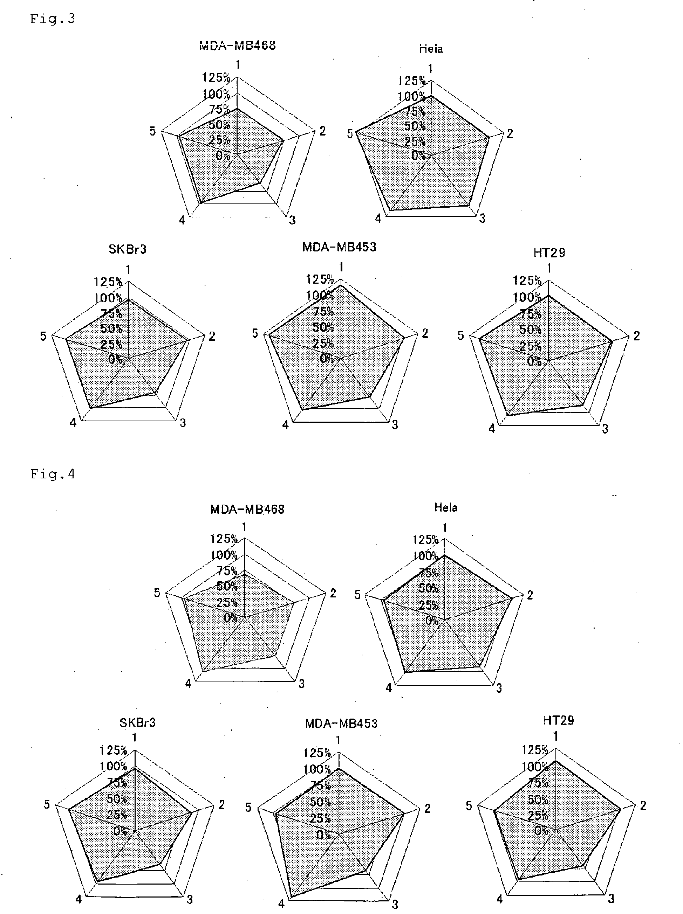

Based on the measurement result, radar charts of Fig 3 were produced. Fig. 3 contains a radar chart in the case of use of the sample for reaction MB453, a radar chart in the case of use of the sample for reaction MB468, a radar chart in the case of use of the sample for reaction SKBr3, a radar chart in the case of use of the sample for reaction Hela, and a radar chart in the case of use of the sample for reaction HT29. These radar charts show a proportion of a measured value obtained by treating the sample for reaction with an inhibitor, letting a measured value when the sample for reaction was not treated with an inhibitor to be 100%. In radar charts, 1 is the case where the sample for reaction was treated with PD153035, 2 is the case where the sample for reaction was treated with AG1478, 3 is the case where the sample for reaction was treated with 4557W, 4 is the case where the sample for reaction was treated with PDGF Receptor Tyrosine Kinase Inhibitor III, and 5 is the case where the sample for reaction was treated with VEGF Receptor Tyrosine Kinase Inhibitor III.

From Fig. 3, the effect of inhibiting the activity of a receptor tyrosine kinase group derived from a cell membrane of each cultured cell could be confirmed. For example, in MDA-MB468, the activity of the receptor tyrosine kinase group was inhibited with PD153035, AG1478 and 4557W. In Hela, in any of inhibitors, inhibition of the receptor tyrosine kinase group was not recognized. In SKBr3, MDA-MB453 and HT29, the activity of the receptor tyrosine kinase group was inhibited with 4557W.

In addition, in Fig. 3, the effect of inhibiting the activity of the receptor tyrosine kinase group with five kinds of inhibitors was recognized for similar characteristic in MB453, SKBr3 and HT29. On the other hand, in MB468 and Hela, distinct characteristics were recognized, respectively.

It is known that receptor tyrosine kinase plays an important role in proliferation of a cell. Therefore, it was predicted that influence of an inhibitor is seen also in proliferation of a cell. Then, in the following Comparative Example 1, influence of an inhibitor on proliferation of a cell was confirmed.

### Comparative Example 1

### Influence of ATP competitive tyrosine kinase inhibitor on proliferation of cell

Regarding five kinds of cultured cells used in Example 1 (MDA-MB453, MDA-MB468, SKBr3, Hela and HT29), influence of five kinds of inhibitors used in Example 1 on cell proliferation was confirmed utilizing the known method.

### (Treatment with inhibitor and cell culturing)

Cells were seeded on each well of a plate for culturing (96 Well Solid White Flat Bottom Polystyrene TC-Treated Microplates, Corning) at 1000 cells per well, and cultured at 37°C for 24 hours. After culturing, an inhibitor was added to each well, and cells were further cultured at 37°C for 3 days. As cells, five kinds of cultured cells used in Example 1 (MDA-MB453, MDA-MB468, SKBr3, Hela and HT29) were used. As the inhibitor, five kinds of inhibitors used in Example 1 (PD153035, AG1478, 4557W, PDGF Receptor Tyrosine Kinase Inhibitor III and VEGF Receptor Tyrosine Kinase Inhibitor III) were used. Concentrations of respective inhibitors are as shown in Table 1. In Table 1, the final concentration is a concentration when the inhibitor is added to a well, and is mixed with cells.

**Table 1**

| Inhibitor | Final concentration (M) |
|---|---|
| PD153035 | 1×10⁻⁶ |
| AG1478 | 1×10⁻⁶ |
| 4557W | 1×10⁻⁵ |
| PDGF Receptor Tyrosine Kinase Inhibitor III | 1×10⁻⁶ |
| VEGF Receptor Tyrosine Kinase Inhibitor III | 1×10⁻⁵ |

### (Measurement of cell proliferation)

For measuring cell proliferation, CellTiter-Glo Luminescent Cell Viability Assay (Promega) was used. This is a reagent kit which can quantitate ATP derived from a cell having the metabolism activity in a medium to measure a viable cell. First, according to a protocol attached to the kit, a measurement reagent was prepared. The measurement reagent was added at 100 µl per well, and a culturing plate was stirred with a shaker for 2 minutes. After stirring, the plate was allowed to stand for 10 minutes, and fluorescence was measured using GENios (TECAN) . Since fluorescence is generated in proportion with an amount of ATP derived from a cell having the metabolism activity in a medium, a viable cell in a medium can be measured by measuring fluorescence.

### (Result)

Based on the measurement result, radar charts of Fig 4 were produced. Fig. 4 contains a radar chart in the case of use of MDA-MB453, a radar chart in the case of use of MDA-MB468, a radar chart in the case of use of SKBr3, a radar chart in the case of use of Hela, and a radar chart in the case of use of HT29. These radar charts show a proportion of a measured value obtained by treating a cell with an inhibitor, letting a measured value when a cell was not treated with an inhibitor to be 100%. In radar charts, 1 is the case where a cell was treated with PD153035, 2 is the case where a cell was treated with AG1478, 3 is the case where a cell was treated with 4557W, 4 is the case where a cell was treated with PDGF Receptor Tyrosine Kinase Inhibitor III, and 5 is the case where a cell was treated with VEGF Receptor Tyrosine Kinase Inhibitor III.

From Fig. 4, the effect of inhibiting proliferation of each cultured cell with five kinds of inhibitors could be confirmed. For example, in MDA-MB468, inhibition of proliferation with PD153035, AG1478 and 4557W was recognized. In Hela, in any of inhibitors, inhibition of proliferation was not recognized. In SKBr3, MDA-MB453 and HT29, inhibition of proliferation with 4557W was recognized.

And, by comparing the result obtained in Example 1 (Fig 3) and the result obtained in Comparative Example 1 (Fig 4), it was seen that there is high correlation between the effect of inhibiting the activity of a receptor tyrosine kinase group with an inhibitor, and the effect of inhibiting proliferation of a cell with an inhibitor. From this, it was seen that it is possible to assess the effect of inhibiting proliferation of a cell with an inhibitor from an activity value of a receptor tyrosine kinase group.

In addition, in the method of Comparative Example 1, in order to confirm inhibition of cell proliferation, cell culturing is required after treatment of a cell with an inhibitor. However, cell culturing is accompanied with troublesome working, and is time-consuming. Furthermore, it is not easy to actually culture a cell taken from a patient. Therefore, it is thought that the method of the present embodiment requiring no culturing working is very useful when the effect of inhibiting proliferation of a cell with an inhibitor is assessed.

In addition, also in Fig. 4 as in Fig. 3, the effect of inhibiting proliferation of a cell with five kinds of inhibitors was recognized in similar characteristic in the sample for reaction MB453, the sample for reaction SKBr3 and the sample for reaction HT29. On the other hand, distinct characteristics were recognized, respectively, in the sample for reaction MB468 and the sample for reaction Hela.

Receptor tyrosine kinase uptakes ATP into an ATP-binding site of an intracellular domain, and transfers a phosphate group of ATP to a substrate. Since a structure of the ATP-binding site is different depending on a kind of receptor tyrosine kinase, a variety of ATP competitive tyrosine kinase inhibitors have been developed as drugs utilizing their characteristics. ATP competitive tyrosine kinase inhibitors used in Example 1 have a common fundamental skeleton and an arm structure of a different structure, as shown in Fig. 2. And, in Example 1, it could be confirmed that the inhibiting effect is different depending on a difference in this arm structure. Like this, it was demonstrated that, based on an activity value of the receptor tyrosine kinase group treated with an inhibitor, it is possible to screen an inhibitor by predicting what an arm structure is possessed by an inhibitor exhibiting the inhibiting effect on the receptor tyrosine group present in a cell membrane of a cell. From this, it was demonstrated that, based on an activity value of the receptor tyrosine kinase group treated with a compound, it is possible to screen a compound by predicting what a structure is possessed by a compound having exhibiting the inhibiting effect on the receptor tyrosine kinase group present in a cell membrane of a cell. In addition, it was demonstrated from results of Example 1 and Comparative Example 1 that, based on an activity value of the receptor tyrosine kinase group treated with an inhibitor, it is possible to screen an inhibitor by predicting what an arm structure is possessed by an inhibitor exhibiting the inhibiting effect on proliferation of a cell. From this, it was demonstrated that, based on an activity value of the receptor tyrosine kinase group treated with a compound, it is possible to screen a compound by predicting what a structure is possessed by a compound exhibiting the inhibiting effect on proliferation of a cell.

### Comparative Example 2

### Influence of ATP competitive tyrosine kinase inhibitor on proliferation of tumor cell in biological body

Influence of the inhibitor used in Example 1 (AG1478 and 4557W) on proliferation of a tumor cell in a mouse body was confirmed.

### (Formation of tumor in mouse body)

MDA-MB468 used in Example 1 was cultured in a culturing solution (DMEM-F12 (Sigma) containing 10% FBS (Hyclone)) so that it became 60% confluent in a 225 cm² flask. The resulting cultured cells were suspended to about 1×10⁷ in 100 µl of DMEM-F12, to prepare a MDA-MB468 cell solution.

A fat pad of a 10-week old female mouse (BALB/c nu/nu) was injected with 100 µl of the MDA-MB468 cell solution. After 14 days, it was confirmed that a tumor became large in a mouse body. Like this, a tumor was formed in three mice bodies.

### (Treatment with inhibitor)

Fourteen days after injection with the MDA-MB468 cell solution, a mouse in which a tumor had been generated was injected with an inhibitor solution. Specifically, AG1478 used in Example 1 was dissolved in 100 µl of dimethyl sulfoxide (DMSO, Sigma) to prepare an inhibitor solution 1. And, the inhibitor solution 1 was injected into a mouse to a dose of the inhibitor of 30 mg/kg/day. Injection with the inhibitor solution 1 was continuously performed for 7 days.

Separately, 4557W used in Example 1 was dissolved in DMSO to prepare an inhibitor solution 2. And, as in the inhibitor solution 1, the inhibitor solution 2 was injected into another mouse.

Further, for comparison, as in the inhibitor solution 1, DMSO was injected into another mouse.

### (Measurement of size of tumor)

The day when the mouse was first injected with an inhibitor solution was regarded as the first day after injection. And, on the first day, the third day, the fifth day, and the eighth day after injection, a volume of a tumor in a mouse body was measured. As a volume of a tumor, a long diameter and a short diameter of a tumor were measured, and a volume was calculated from a long diameter and a short diameter on the assumption that a shape of a tumor was an elliptic sphere.

Also regarding the mouse injected with DMSO, a volume of a tumor was measured as described above.

### (Result)

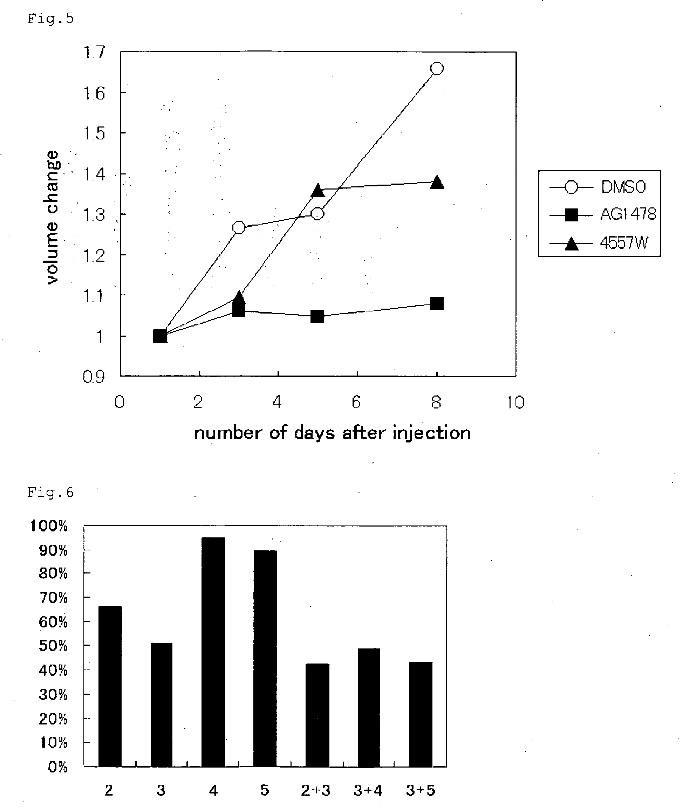

The measurement result is shown in Fig. 5. Fig. 5 shows a change in a volume on the third day, the fifth day and eighth day, letting a volume on the first day after injection to be 1.

From Fig. 5, the effect of inhibiting proliferation of a tumor cell in a mouse body with an inhibitor could be confirmed. For example, when a change in a volume of a tumor of the mouse injected with DMSO, and a change in a volume of a tumor of the mouse injected with an inhibitor were compared, inhibition of proliferation of a tumor cell with AG1478 and 4557W was recognized. And, the result of Comparative Example 2 was consistent with the result of Example 1 . That is, regarding AG1478 and 4557W by which the effect of inhibiting the activity of the receptor tyrosine kinase group of MDA-MD468 was confirmed in Example 1, inhibition of proliferation of a tumor cell in a mouse body was recognized also in Comparative Example 2. From this, it was seen that it is possible to assess the effect of inhibiting proliferation of a tumor cell in a biological body with an inhibitor, from an activity value of the receptor tyrosine kinase group.

### Example 2

### Influence in the case of combination of two kinds of ATP competitive tyrosine kinase inhibitors

In the present Example, the sample for reaction MB468 used in Example 1 was treated with a combination of two kinds of ATP competitive tyrosine kinase inhibitors. Using the GST-poly(Glu, Tyr) substrate, an activity value of the receptor tyrosine kinase group contained in the treated sample for reaction MB468 was measured. Based on the resulting activity value, an extent of inhibition of the receptor tyrosine kinase activity of a cultured cell by a combination of two kinds of inhibitors was studied.

### (Sample for reaction)

The sample for reaction MB468 prepared in Example 1 was used.

### (ELISA plate)

The plate for ELISA prepared in Example 1 was used in the following enzyme reaction.

### (Treatment with ATP competitive tyrosine kinase inhibitor)

In the present Example, among ATP competitive tyrosine kinase inhibitors used in Example 1, four kinds of inhibitors of AG1478, 4557W, PDGF Receptor Tyrosine Kinase Inhibitor III and VEGF Receptor Tyrosine Kinase Inhibitor III were used. When two kinds of inhibitors were combined, AG1478 and 4557W, 4557W and PDGF Receptor Tyrosine Kinase Inhibitor III, and 4557W and VEGF Receptor Tyrosine Kinase Inhibitor III were used, respectively, by combining them.

First, 25 µl of the sample for reaction MB468 was dispensed into eight tubes, respectively. Among eight tubes, to the first tube was added 25 µl of a treating solution containing 400 µl of AG1478 (20 mM HEPES pH7.4, 20 mM MnCl₂, 2 mM DTT, 1%NP40, 10% glycerol, 200 µM Na₃VO₄, 50 mM NaF, 400 µM ATP). To the second tube was added 25 µl of a treating solution containing 400 µM of 4557W. To the third tube was added 25 µl of a treating solution containing 400 µM PDGF Receptor Tyrosine Kinase Inhibitor III. To the fourth tube was added 25 µl of a treating solution containing 400 µM VEGF Receptor Tyrosine Kinase Inhibitor III. To the fifth tube was added 25 µl of a treating solution containing 400 µM AG1478 and 400 µM 4557W. To the sixth tube was added 25 µl of a treating solution containing 400 µM 4557W and 400 µM PDGF Receptor Tyrosine Kinase Inhibitor III. To the seventh tube was added 25 µl of a treating solution containing 400 µM 4557W and 400 µM VEGF Receptor Tyrosine Kinase Inhibitor III. To the eighth tube was added 25 µl of a treating solution containing no inhibitor. The thus obtained solutions are used as a reaction solution. In addition, in order to suppress the enzyme activity of receptor tyrosine kinase, this working was performed under the condition of not higher than 4°C.

### (Enzyme reaction and detection of phosphorylated substrate)

According to the same manner as that of Example 1, each reaction solution was measured for phosphorylation of a substrate by an ELISA method.

### (Results)

The measurement result is shown in Fig. 6. Fig. 6 shows a proportion of a measured value obtained by treating the sample for reaction MB468 with an inhibitor, letting a measurement value when the sample for reaction MB468 was not treated with an inhibitor to be 100%. In the figure, 2 is the case where the sample for reaction ME468 was treated with AG1478, 3 is the case where the sample for reaction MB468 was treated with 4557W, 4 is the case where the sample for reaction MB468 was treated with PDGF Receptor Tyrosine Kinase Inhibitor III, 5 is the case where the sample for reaction MB468 was treated with VEGF Receptor Tyrosine Kinase Inhibitor III, 2+3 is the case where the sample for reaction MB468 was treated with a combination of AG1478 and 4557W, 3+4 is the case where the sample for reaction MB468 was treated with a combination of 4557W and PDGF Receptor Tyrosine Kinase Inhibitor III, and 3+5 is the case where the sample for reaction M468 was treated with a combination of 4557W and VEGF Receptor Tyrosine Kinase Inhibitor III.

From Fig. 6, it was seen that use of inhibitors by combining them rather than use of them alone exhibits the higher effect of inhibiting the activity of a receptor tyrosine kinase group. The inhibitor which alone exhibited the highest activity inhibiting effect is 4557W. When this 4557W and another inhibitor were combined, the higher activity inhibiting effect was exhibited than 4557W alone. Particularly, PDGF Receptor Tyrosine Kinase Inhibitor III and VEGF Receptor Tyrosine Kinase Inhibitor III alone exhibit little effect of inhibiting the activity of receptor tyrosine kinase. However, by combining with 4557W, the high activity inhibiting effect was exhibited.

It was predicted that the similar result is obtained also in inhibition of proliferation of a cell. Then, inhibition of proliferation of a cell with an inhibitor was confirmed in the following Comparative Example 3.

### Comparative Example 3

Influence of combination of two kinds of ATP competitive tyrosine kinase inhibitors on cell proliferation

Regarding the cultured cell MDA-MB468, influence of each inhibitor used in Example 2 on cell proliferation was confirmed utilizing the known method.

### (Treatment with inhibitor and cell culturing)

According to the same manner as that of Comparative Example 1, after MDA-MB468 was treated with an inhibitor in a well of a culturing plate, cells were cultured. As the inhibitor, among ATP competitive tyrosine kinase inhibitors used in Example 2, four kinds of inhibitors (AG1478, 4557W, PDGF Receptor Tyrosine Kinase Inhibitor III and VEGF Receptor Tyrosine Kinase Inhibitor III) were used. When two kinds of inhibitors were combined, AG1478 and 4557W, 4557W and PDGF Receptor Tyrosine Kinase Inhibitor III, and 4557W and VEGF Receptor Tyrosine Kinase Inhibitor III were used, respectively, by combining them. Concentrations of respective inhibitors are as shown in Table 2. The final concentration in Table 2 is a concentration when the inhibitor was added to a well, and was mixed with MDA-MB468.

**Table 2**

| Inhibitor | Final concentration (M) |
|---|---|
| AG1478 | 1×10⁻⁶ |
| 4557W | 1×10⁻⁵ |
| PDGF Receptor Tyrosine Kinase Inhibitor III | 1×10⁻⁶ |
| VEGF Receptor Tyrosine Kinase Inhibitor III | 1×10⁻⁵ |
| AG1478 | 1×10⁻⁶ |
| 4557W | 1×10⁻⁵ |
| 4557W | 1×10⁻⁵ |
| PDGF Receptor Tyrosine Kinase Inhibitor III | 1×10⁻⁶ |
| 4557W | 1×10⁻⁵ |
| VEGF Receptor Tyrosine Kinase Inhibitor III | 1×10⁻⁵ |

### (Measurement of cell proliferation)

According to the same manner as that of Comparative Example 1, cell proliferation was measured.

### (Results)

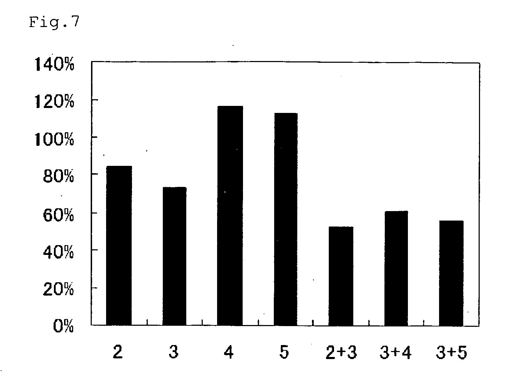

The measurement result is shown in Fig. 7. Fig. 7 shows a proportion of a measured value obtained by treating MDA-MB468 with the inhibitor, letting a measured value when MDA-MB468 was not treated with the inhibitor to be 100%. In the figure, 2 is the case where MDA-MB468 was treated with AG1478, 3 is the case where MDA-MB468 was treated with 4557W, 4 is the case where MDA-MB468 was treated with PDGF Receptor Tyrosine Kinase Inhibitor III, 5 is the case where MDA-MB468 was treated with VEGF Receptor Tyrosine Kinase Inhibitor III, 2+3 is the case where MDA-MB468 was treated with a combination of AG1478 and 4557W, 3+4 is the case where MDA-MB468 was treated with a combination of 4557W and PDGF Receptor Tyrosine Kinase Inhibitor III, and 3+5 is the case where MDA-MB468 was treated with a combination of 4557W and VEGF Receptor Tyrosine Kinase Inhibitor III.

By comparing the result obtained in Example 2 (Fig. 6) with the result obtained in Comparative Example 3 (Fig. 7), it was seen that there is high correlation between the effect of inhibiting the activity of receptor tyrosine kinase with the inhibitor, and the effect of inhibiting proliferation of a cell with the inhibitor, also in the case of combination of two kinds of inhibitors. From this, it was seen that it is possible to assess the effect of inhibiting proliferation of a cell with a combination of a plurality of inhibitors, from an activity value of the receptor tyrosine kinase group.

As seen also from the results of Example 2 and Comparative Example 3, it is very difficult to predict the synergistic effect of the inhibitor when a plurality of inhibitors are used, from the inhibiting effect when inhibitors are used alone. Therefore, when a combination of a plurality of inhibitors is studied, it is necessary to actually confirm the inhibiting effect. For example, as in Comparative Example 3, in order to confirm the effect of inhibiting proliferation of a cell, cell culturing is required in many cases after treatment of a cell with a plurality of inhibitors. However, cell culturing is accompanied with troublesome working, and is time-consuming. Furthermore, it is not easy to actually culture a cell taken from a patient. Therefore, it is thought that the method of the present embodiment requiring no culturing working is very useful, when a combination of a plurality of inhibitors is studied.

## Claims

1. A method for assessing the proliferation inhibiting effect of a receptor tyrosine kinase inhibitor on a tumor cell, by determining the activity value of the group of receptor tyrosine kinases in a sample of said tumor cell, comprising the steps of:
- preparing a sample of said tumor cell comprising a membrane fraction, by separating a cytoplasm from a tumor cell, wherein the sample contains the group of receptor tyrosine kinases;
- separating the sample into first and second samples;
- treating the first sample with the inhibitor;
- contacting each of the first and second samples with a universal receptor tyrosine kinase substrate, wherein the universal substrate comprises a poly (Glu:Tyr) (4:1) peptide, a poly (Glu:Tyr) (1:1) peptide, a poly (Glu:Tyr:Ala) (6:1:3) peptide, a poly (Glu:Tyr:Ala) (1:1:1) peptide, or a poly (Glu:Tyr:Ala:Lys) (2:1:6:5) peptide;
- detecting the substrate phosphorylated by the group of receptor tyrosine kinases in each of the first and second samples;
- measuring a first activity value of the group of receptor tyrosine kinases in the first sample and a second activity value of the group of receptor tyrosine kinases in the second sample based on the detection results; and
- assessing the proliferation inhibiting effect of the inhibitor based on the difference between said first and second activity values of the group of receptor tyrosine kinases.

2. The method according to claim 1, wherein the assessing step is performed by
- calculating a difference between the first activity value and the second activity value,
- comparing the calculated difference with a threshold, and
- judging that the proliferation inhibiting effect is high when the difference is not less than the threshold.

3. The method according to claim 1, wherein the assessing step is performed by
- calculating a proportion between the first activity value and the second activity value,
- comparing the calculated proportion with a threshold, and
- judging that the proliferation inhibiting effect is high when the proportion is lower than the threshold.

4. The method according to any one of claims 1 to 3, wherein the preparing step is performed by
- fragmentating the tumor cell in a buffer solution,
- mixing the fragmentated tumor cell and a solution containing a surfactant, and
- collecting a supernatant of the resulting mixture as the sample containing various receptor tyrosine kinases.

5. The method according to claim 4, wherein the surfactant is a nonionic surfactant.

6. The method according to any one of claims 1 to 5, wherein the receptor tyrosine kinases contain insulin-like growth factor receptor (IGFR), platelet-derived growth factor receptor (PDGFR), human epithelial growth factor receptor (HER) or vascular endothelial growth factor (VEGFR).

7. The method according to any one of claims 1 to 6, wherein the inhibitor inhibits the activity of receptor tyrosine kinase by binding to an ATP-binding site of receptor tyrosine kinase.

8. A method for screening a compound which inhibits proliferation of a tumor cell, comprising the steps of:
- preparing a sample comprising a membrane fraction by separating a cytoplasm from the tumor cell, wherein the sample contains the group of receptor tyrosine kinases;
- separating the sample into first and second samples;
- treating the first sample with a compound;
- contacting each of the first and second samples with a universal substrate for receptor tyrosine kinases, wherein said universal substrate comprises a poly (Glu:Tyr) (4:1) peptide, a poly (Glu:Tyr) (1:1) peptide, a poly (Glu:Tyr:Ala) (6:1:3) peptide, a poly (Glu:Tyr:Ala) (1:1:1) peptide, or a poly (Glu:Tyr:Ala:Lys) (2:1:6:5) peptide;
- detecting the substrate phosphorylated by the group of receptor tyrosine kinases in each of the first and second samples;
- measuring first activity value of the group of receptor tyrosine kinases in the first sample and a second activity value of the group of receptor tyrosine kinase in the second sample; and
- screening a compound which inhibits proliferation of the tumor cell, based on the first and second activity values.

9. The method according to any one of claims 1 to 8, wherein the universal substrate comprises a poly (Glu:Tyr) (4:1) peptide.

10. The method according to claim 9, wherein the universal substrate comprises a peptide as defined in SEQ ID NO.1.

11. The method according to claim 10, wherein the universal substrate is the protein as defined in SEQ ID NO.5.

## Patentansprüche

1. Verfahren zum Beurteilen der proliferationshemmenden Wirkung eines Rezeptor-Ttyrosinkinase-Inhibitors an einer Tumorzelle durch Bestimmen des Aktivitätswertes von der Gruppe der Tyrosinkinasen in einer Probe der Tumorzelle, das die Schritte umfasst:
- Herstellen einer Probe der Tumorzelle, die eine Membranfraktion umfasst, durch Abtrennen eines Zytoplasmas von einer Tumorzelle, wobei die Probe die Gruppe der Rezeptor-Tyrosinkinasen enthält;
- Aufteilen der Probe in eine erste und eine zweite Probe;
- Behandeln der ersten Probe mit dem Inhibitor;
- Inkontaktbringen von der ersten und der zweiten Probe mit einem universellen Rezeptor-Tyrosinkinase-Substrat, wobei das universelle Substrat ein Poly(Glu:Tyr)-(1:1)-peptid, ein Poly(Glu:Tyr:Ala)-(6:1:3)-peptid, ein Poly-(Glu:Tyr:Ala)-(1:1:1)-peptid oder ein Poly(Glu:Tyr: Ala:Lys)-(2:1:6:5)-peptid umfasst;
- Nachweisen des durch die Gruppe der Rezeptor-Tyrosinkinasen in der ersten und in der zweiten Probe phosphorylierten Substrats;
- Messen eines ersten Aktivitätswertes von der Gruppe der Rezeptor-Tyrosinkinasen in der ersten Probe und eines zweiten Aktivitätswertes von der Gruppe der Rezeptor-Tyrosinkinasen in der zweiten Probe auf der Grundlage der erfassten Ergebnisse; und
- Beurteilen der proliferationshemmenden Wirkung von dem Inhibitor auf der Grundlage der Differenz zwischen dem ersten und dem zweiten Aktivitätswert von der Gruppe der Rezeptor-Tyrosinkinasen.

2. Verfahren nach Anspruch 1, wobei der Beurteilungsschritt durchgeführt wird durch:
- Berechnen einer Differenz zwischen dem ersten Aktivitätswert und dem zweiten Aktivitätswert,
- Vergleichen der berechneten Differenz mit einem Schwellenwert, und
- Bewerten, ob die proliferationshemmende Wirkung hoch ist, wenn die Differenz nicht kleiner als der Schwellenwert ist.

3. Verfahren nach Anspruch 1, wobei der Beurteilungsschritt durchgeführt wird durch:
- Berechnen von einem Verhältnis zwischen dem ersten Aktivitätswert und dem zweiten Aktivitätswert,
- Vergleichen des berechneten Verhältnisses mit einem Schwellenwert, und
- Bewerten, ob die proliferationshemmende Wirkung hoch ist, wenn das Verhältnis nicht kleiner als der Schwellenwert ist.

4. Verfahren nach einem der Ansprüche 1 bis 3, wobei der Herstellungsschritt durchgeführt wird durch:
- Fragmentieren der Tumorzelle in einer Pufferlösung,
- Mischen der fragmentierten Tumorzelle mit einer Lösung, die ein oberflächenaktives Mittel enthält, und
- Sammeln eines Überstandes von der resultierenden Mischung als Probe, die verschiedene Rezeptor-Tyrosinkinasen enthält.

5. Verfahren nach Anspruch 4, wobei das oberflächenaktive Mittel ein nichtionisches Netzmittel ist.

6. Verfahren nach einem der Ansprüche 1 bis 5, wobei die Rezeptor-Tyrosinkinasen den Insulinähnlichen Wachstumsfaktor-Rezeptor (IGFR), den Plättchenwachstumsfaktor-Rezeptor (PDGFR), den humanen epithelialen Wachstumsfaktor-Rezeptor (HER) oder den Vaskulären Endothelialen Wachstumsfaktor-Rezeptor (VEGFR) umfassen.

7. Verfahren nach einem der Ansprüche 1 bis 6, wobei der Inhibitor die Aktivität der Rezeptor-Tyrosinkinase durch Bindung an eine ATP-Bindungsstelle der Rezeptor-Tyrosinkinase hemmt.

8. Verfahren zum Screening einer Verbindung, welche die Proliferation einer Tumorzelle hemmt, das die Schritte umfasst:
- Herstellen einer Probe, die eine Membranfraktion umfasst, durch Abtrennen eines Zytoplasmas von der Tumorzelle, wobei die Probe die Gruppe der Rezeptor-Tyrosinkinasen enthält;
- Aufteilen der Probe in eine erste und eine zweite Probe;
- Behandeln der ersten Probe mit einer Verbindung;
- Inkontaktbringen von der ersten und der zweiten Probe mit einem universellen Substrat für Rezeptor-Tyrosinkinasen, wobei das universelle Substrat ein Poly(Glu:Tyr)-(4:1)-peptid, ein Poly(Glu:Tyr)-(1:1)-peptid, ein Poly(Glu:Tyr:Ala)-(6:1:3)-peptid, ein Poly-(Glu:Tyr:Ala)-(1:1:1)-peptid oder ein Poly(Glu:Tyr:Ala: Lys)-(2:1:6:5)-peptid umfasst;
- Nachweisen des durch die Gruppe der Rezeptor-Tyrosinkinasen in der ersten und in der zweiten Probe phosphorylierten Substrats;
- Messen eines ersten Aktivitätswertes von der Gruppe der Rezeptor-Tyrosinkinasen in der ersten Probe und eines zweiten Aktivitätswertes von der Gruppe der Rezeptor-Tyrosinkinasen in der zweiten Probe; und
- Screening einer Verbindung, welche die Proliferation von der Tumorzelle hemmt, auf der Grundlage des ersten und des zweiten Aktivitätswertes.

9. Verfahren nach einem der Ansprüche 1 bis 8, wobei das universelle Substrat ein Poly(Glu:Tyr)-(4:1)-peptid umfasst.

10. Verfahren nach Anspruch 9, wobei das universelle Substrat ein Peptid umfasst, wie es in SEQ-ID Nr. 1 definiert ist.

11. Verfahren nach Anspruch 10, wobei das universelle Substrat das Protein ist, das in SEQ-ID Nr. 5 definiert ist.

## Revendications

1. Procédé d'évaluation de l'effet d'inhibition de la prolifération d'un inhibiteur de récepteurs tyrosine kinases sur une cellule tumorale, par détermination de la valeur d'activité du groupe de récepteurs tyrosine kinases dans un échantillon de ladite cellule tumorale, qui comprend les étapes consistant à :
- préparer un échantillon de ladite cellule tumorale qui comprend une fraction membranaire, par séparation d'un cytoplasme d'une cellule tumorale, l'échantillon contenant le groupe de récepteurs tyrosine kinases ;
- séparation de l'échantillon en des premier et second échantillons ;
- traitement du premier échantillon avec l'inhibiteur ;
- mise en contact de chacun des premier et second échantillons avec un substrat universel pour récepteurs tyrosine kinases, le substrat universel comprenant un polypeptide (Glu:Tyr) (4:1), un polypeptide (Glu:Tyr) (1:1), un polypeptide (Glu:Tyr: Ala) (6:1:3), un polypeptide (Glu:Tyr:Ala) (1:1:1), ou un polypeptide (Glu:Tyr:Ala:Lys) (2:1:6:5) ;
- détection du substrat phosphorylé par le groupe de récepteurs tyrosine kinases dans chacun des premier et second échantillons ;
- mesure d'une première valeur d'activité du groupe de récepteurs tyrosine kinases dans le premier échantillon et d'une seconde valeur d'activité du groupe de récepteurs tyrosine kinases dans le second échantillon en se basant sur les résultats de détection ; et
- évaluation de l'effet d'inhibition de la prolifération de l'inhibiteur en se basant sur la différence entre lesdites première et seconde valeurs d'activité du groupe de récepteurs tyrosine kinases.

2. Procédé selon la revendication 1, dans lequel l'étape d'évaluation est réalisée par
- calcul d'une différence entre la première valeur d'activité et la seconde valeur d'activité,
- comparaison de la différence calculée à un seuil, et
- estimation que l'effet d'inhibition de la prolifération est élevé lorsque la différence n'est pas inférieure au seuil.

3. Procédé selon la revendication 1, dans lequel l'étape d'évaluation est réalisée par
- calcul d'une proportion entre la première valeur d'activité et la seconde valeur d'activité,
- comparaison de la proportion calculée à un seuil, et
- estimation que l'effet d'inhibition de la prolifération est élevé lorsque la proportion est inférieure au seuil.

4. Procédé selon l'une quelconque des revendications 1 à 3, dans lequel l'étape de préparation est réalisée par
- fragmentation de la cellule tumorale dans une solution tampon,
- mélange de la cellule tumorale fragmentée et d'une solution contenant un surfactant, et
- collecte d'un surnageant du mélange résultant en tant qu'échantillon contenant différents récepteurs tyrosine kinases.

5. Procédé selon la revendication 4, dans lequel le surfactant est un surfactant non ionique.

6. Procédé selon l'une quelconque des revendications 1 à 5, dans lequel les récepteurs tyrosine kinases contiennent le récepteur du facteur de croissance de type insuline (IGFR), le récepteur du facteur de croissance dérivé des plaquettes (PDGFR), le récepteur du facteur de croissance épithélial humain (HER) ou le récepteur du facteur de croissance endothélial vasculaire (VEGFR).

7. Procédé selon l'une quelconque des revendications 1 à 6, dans lequel l'inhibiteur inhibe l'activité du récepteur tyrosine kinase par liaison à un site de liaison de l'ATP du récepteur tyrosine kinase.

8. Procédé de criblage d'un composé qui inhibe la prolifération d'une cellule tumorale, qui comprend les étapes consistant à :
- préparer un échantillon comprenant une fraction membranaire par séparation d'un cytoplasme de la cellule tumorale, l'échantillon contenant le groupe de récepteurs tyrosine kinases ;
- séparer l'échantillon en des premier et second échantillons ;
- traiter le premier échantillon avec un composé ;
- mettre en contact chacun des premier et second échantillons avec un substrat universel pour récepteurs tyrosine kinases, ledit substrat universel comprenant un polypeptide (Glu:Tyr) (4:1), un polypeptide (Glu:Tyr) (1:1), un polypeptide (Glu:Tyr:Ala) (6:1:3), un polypeptide (Glu:Tyr:Ala) (1:1:1), ou un polypeptide (Glu:Tyr:Ala:Lys) (2:1:6:5) ;
- détecter le substrat phosphorylé par le groupe de récepteurs tyrosine kinases dans chacun des premier et second échantillons ;
- mesurer une première valeur d'activité du groupe de récepteurs tyrosine kinases dans le premier échantillon et une seconde valeur d'activité dans le groupe de récepteurs tyrosine kinases dans le second échantillon ; et,
- cribler un composé qui inhibe la prolifération de la cellule tumorale, en se basant sur les première et seconde valeurs d'activité.

9. Procédé selon l'une quelconque des revendications 1 à 8, dans lequel le substrat universel comprend un polypeptide (Glu:Tyr) (4:1).

10. Procédé selon la revendication 9, dans lequel le substrat universel comprend un peptide tel que défini dans SEQ ID NO : 1.

11. Procédé selon la revendication 10, dans lequel le substrat universel est la protéine telle que définie dans SEQ ID NO : 5.


### SEQUENCE LISTING

<110> Sysmex corporation
<120> METHOD FOR ASSESSING PROLIFERATION INHIBITING EFFECT OF INHIBITOR, AND METHOD FOR DETERMINING SENSITIVITY OF TUMOR CELL TO INHIBITOR
<130> SYSM-015-EP
<150> JP 2006-261699
   <151> 2006-09-27
<150> JP 2007-224923
   <151> 2007-08-30
<160> 5
<170> PatentIn version 3.1
<210> 1
   <211> 25
   <212> PRT
   <213> Artificial sequence
<220>
   <223> Amino acid sequence of poly(Glu,Tyr) peputide
<400> 1
   Glu Glu Glu Glu Tyr Glu Glu Glu Glu Tyr Glu Glu Glu Glu Tyr Glu
   1               5                   10                  15
   Glu Glu Glu Tyr Glu Glu Glu Glu Tyr
               20                  25
<210> 2
   <211> 75
   <212> DNA
   <213> Artificial sequence
<220>
   <223> Designed DNA based on amino acid sequence of poly(Glu,Tyr) peptid e
<400> 2
   gaggaggagg agtacgaaga agaagaatat gaagaagaag aatatgaaga agaagaatat      60
   gaggaggaag agtac                                                       75
<210> 3
   <211> 25
   <212> DNA
   <213> Artificial sequence
<220>
   <223> Designed DNA based on nucleotide sequence of poly(Glu,Tyr) peptid e
<400> 3
   ccggggatcc gaggaggagg agtac 25
<210> 4
   <211> 27
   <212> DNA
   <213> Artificial sequence
<220>
   <223> Designed DNA based on nucleotide sequence of poly(Glu,Tyr) peptid e
<400> 4
   cccggggaat tcgtactctt cttcctc 27
<210> 5
   <211> 263
   <212> PRT
   <213> Artificial sequence
<220>
   <223> Amino acid sequence of GST-poly(Glu,Tyr) fusion protein
<400> 5
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Leu Val Pro Arg
       210                 215                 220
   Gly Ser Glu Glu Glu Glu Tyr Glu Glu Glu Glu Tyr Glu Glu Glu Glu
   225                 230                 235                 240
   Tyr Glu Glu Glu Glu Tyr Glu Glu Glu Glu Tyr Glu Phe Pro Gly Arg
                   245                 250                 255
   Leu Glu Arg Pro His Arg Asp
               260